# EUROPEAN PATENT APPLICATION

(11) **EP 2 749 300 A1**
(43) Date of publication of application: **02.07.2014**
(21) Application number: 12382534.1
(22) Date of filing: 26.12.2012
(51) Int. Cl.: A61L 27/18, A61L 31/06, C08G 65/48, C08L 71/12

(54) **Modified polyaryletherketone polymer (PAEK) and process to obtain it**

(71) Applicant: Universidad Del Pais Vasco-Euskal Herriko Unibertsitatea, 48170 Zamudio (Vizcaya) (ES); FUNDACIÓN TECNALIA RESEARCH & INNOVATION, 20009 Donostia-San Sebastian (ES); Centro de Investigación Biomedica en Red en Bioingenieria, Biomateriales y Nanomedicina, 50018 Zaragoza (ES)
(72) Inventor: Aizpurua Iparraguirre, Jesús, Mª, 48170 ZAMUDIO (Vizcaya) (ES); Sagartzazu Aizpurua, Maialen, 48170 ZAMUDIO (Vizcaya) (ES); Braceras Izaguirre, Iñigo, 20009 DONOSTIA- SAN SEBASTIAN (ES); Azpiroz Dorronsoro, Francisco, Javier, 20009 DONOSTIA- SAN SEBASTIAN (ES); Oyarbide Vicuña, Joseba, 50018 ZARAGOZA (ES)
(74) Representative: Carpintero Lopez, Francisco

(57) **Abstract**

The present invention relates to a modified polyaryletherketone polymer (PAEK) with chemically modified surfaces with azides, alkynes, thiols, maleimides, sulfonylazides or thio acids, suitable for "click" reactions and to a process to obtain it. It also relates to the conjugated biomaterials derived thereof, PAEK type materials with surfaces modified with a RGD (Arg- Gly-Asp) and/or OGP₁₀₋₁₄ (Tyr-Gly-Phe-Gly-Gly) peptidomimetics and to a process to obtain it. These materials are particularly useful for manufacturing medical devices. Finally, the present invention also relates to a fluorescent PAEK material.

## Description

### FIELD OF THE INVENTION

The present invention relates to a modified polyaryletherketone polymer (PAEK) with chemically modified surfaces with azides, alkynes, thiols, maleimides, sulfonylazides or thio acids, suitable for "click" reactions and to a process to obtain it.

It also relates to the conjugated biomaterials derived thereof, PAEK type materials with surfaces modified with a RGD (Arg- Gly-Asp) and/or OGP₁₀₋₁₄ (Tyr-Gly-Phe-Gly-Gly) peptidomimetics and to a process to obtain it.

These materials are particularly useful for manufacturing medical devices or tissue engineering or cell culture matrices.

Finally, the present invention also relates to a fluorescent PAEK material.

### BACKGROUND ART

PAEK is a thermoplastic with acceptable biocompatibility that can be processed in fibers, films, coatings, matrices or porous materials and has been used in the manufacturing of implants and prosthetic devices.

However, as PAEK shows poor tissue integration properties (e.g. osseointegration), more advanced medical applications require an active role of the biomaterial to promote the adhesion, proliferation and/or differentiation of specific cells.

Therefore, it is highly desirable to develop new modified PAEK and the methods to modify the surface of PAEK implants. In this way, the excellent mechanical, chemical and structural properties of the PAEK polymer are preserved in the bulk material, while different physical or biological properties are conferred by the components grafted to the surface.

Polyetheretherketone, commonly referred to as PEEK, is a member of the PAEK polymer family.

Surface chemical modification of PEEK (poly ether ether ketone) is known to occur by addition and condensation reactions to the ketone carbonyl group under rather harsh reaction conditions. These modifications, followed by conventional carbodiimide- or N-hydroxysuccinimide-activated amide bond formation, can be used to attach peptides to PEEK films (Devine JN et al. WO 2002000763 A1 "Bio-compatible surface functionalized polyetherketone materials, their manufacture and use"). On the other hand, carbonyl modification reactions of PEEK polymers are strongly dependent on crystallinity. PEEK polymers have been reacted with hydroxylamine to form the corresponding PEEK-oxime and this material has been derivatized with the very reactive benzenesulfonylisocyanate to provide the corresponding phenylsulfonyl carbamate (Franchina NL et al. Macromolecules, 1991, 24: 3045-3049 "Surface modifications of poly (ether ether ketone").

Chemical transformations at the surface of PEEK polymers, can be monitored preferably by contact angle, X-ray photoelectron spectroscopy (XPS), fluorescence determination after derivatization with suitable light-responsive agents (e.g. dansyl group) or enzyme-linked immunosorbent assays (ELISA).

Conjugation of mixtures of multifunctional bioactive molecules to PEEK polymers using conventional amide chemistry is a nonobvious task when the protecting group approach is applied. Alternatively, copper-catalyzed bio-orthogonal ("click") chemistry provides another route to the surface modification of polymer materials with suitably modified biomolecules (Ofir, Y. et al. US 2009264317 A1 "Functionalized nanostructure, methods of manufacture thereof and articles comprising the same" see also: Nebhani, L. et al. Adv. Mater. 2009, 21: 3442-3468 "Orthogonal transformations on solid substrates: efficient avenues to surface modification"), but the porous or rough surface of some polymeric materials or grafted peptides may adsorb significant amounts of toxic copper salts, which is often needed to promote orthogonal conjugation. This might render the resulting functionalized polymer materials (e.g. porous or very rough PEEK) unacceptable for applications in medicine. Copper-free methods to perform the azide-alkyne cycloaddition under mild conditions are known in the art (Lutz, J-F Angew Chem Int Ed 2008 47: 2182-2184 "Copper-free azide-alkyne cycloadditions: new insights and perspectives"). No method to achieve bio-orthogonal peptide grafting to the surface of PEEK-type polymers is known in the art.

Osteoblast cell adhesion to prosthetic devices has been improved using Arg-Gly-Asp (RGD) peptidomimetics (Kessler H et al. Biomaterials 2003 24: 4385-4415 "RGD modified polymers: biomaterials for stimulated cell adhesion and beyond"). This tripeptide or some related peptidomimetic surrogates have been used to regulate the interaction with the extracellular matrix of cell membranes and to improve cell function around endosseous or bone implants, as shown for example in Kantlehner M et al. ChemBioChem 2000 1: 107-114 "Surface coating with cyclic RGD peptides stimulates osteoblast adhesion and proliferation as well as bone formation"; see also: Biltresse S. et al. Biomaterials 2005 26: 4576-4587 "Cell adhesive PET membranes by surface grafting of RGD peptidomimetics".

Another peptide which has shown to promote cell proliferation is the osteogenic growth peptide (OGP), a short naturally occurring 14-mer growth factor peptide found in serum. As a soluble peptide, OGP regulates proliferation, differentiation and matrix mineralization in osteoblast lineage cells. Studies have demonstrated sensitivity of osteoblast lineage cells to changes in exogenous concentrations of OGP and the effectiveness of immobilized OGP incorporated into biomaterials is also known in the art, (Moore et al. Biomaterials 2010, 31: 1604-1611).

However, RGD and OGP peptides or their cyclic surrogates are prone to a fast deactivation by proteolytic cleavage, leading to lowered serum concentrations in systemic applications or inactive surfaces when grafted onto biomaterials.

In view of the state of the art, it still persist the need for PAEK type materials possessing good mechanical properties that can integrate with bone tissue. Particularly suitable are PEEK type implantable materials, either compact or with controlled pore sizes, chemically functionalized in their surface with bio-orthogonal groups like azide, terminal alkyne, cyclooctyne, maleimido, thiol, suitable for conjugation with unprotected peptides, proteins, saccharides, etc under mild and selective conditions. Very suitable are also the former PEEK type materials covalently functionalized at their surface with nonhydrolyzable RGD and/or OGP₁₀₋₁₄ peptidomimetics to endow long term osteoinductive properties to the polymer. Finally, a metal-free method allowing the one-step chemical polyfunctionalization at the surface of PEEK type materials with several mimetics at the same time in variable proportions is specially suited for the manufacturing of medical implants for human and animal therapy.

### SUMMARY OF THE INVENTION

Polyaryletherketone polymers (PAEK) are characterized by aryl rings that are linked via oxygen bridges (ethers) and carbonyl groups (ketones).

The inventors have developed a modified polyaryletherketone polymer (PAEK) with a linker endowed with latent conjugation reactivity ("click"). This linker with latent conjugation reactivity ("click") is advantageous, since it is possible to incorporate biologically active components, labelling components or other compounds to the surface of the material with a click reaction. Moreover, they provide the possibility to maintain a sustained functionality along time.

Therefore, a first aspect of the invention refers to a modified polyaryletherketone polymer (PAEK) comprising aromatic rings with ether and ketones linkages in the backbone, wherein the aromatic ring is a substituted or unsubstituted phenylene as represented by the formula (I); 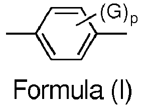 wherein G is independently in each occurrence: hydrogen, a C₁₋₄ alkyl or a halogen; more preferably G is: hydrogen, methyl, ethyl, chlorine, bromine, or fluorine; p is an integer between 0 and 4 inclusive; characterized in that in said PAEK polymer at least one ketone group is replaced by C=N-O-(R¹)-X wherein:
R¹ is a biradical consisting of C₁₋₂₀ alkylene; optionally substituted with one or more C₁₋₄ alkyl groups; optionally containing -C=C- bonds; optionally containing -C≡C-bonds; in which 0, 1, 2, 3, 4, 5 or 6 -CH₂- groups are optionally replaced by groups selected from: -O-, -S-, -N(H)-, -N(C₁₋₄ alkyl)-, -CO-, -C(O)O-, - C(O)NH-, -C(O)N(C₁₋₄ alkyl)-, -NHC(O)NH- or -NHC(O)O- and;
X is a group selected from: N-maleimide, N-maleimide-furan cycloadduct, thiol, thio acid, azide, sulfonylazide, ethynyl, iodoethynyl or an activated cyclooctynyl group represented by the formulae:

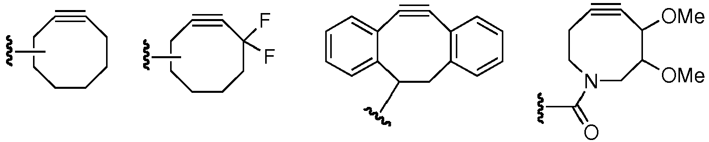

The second aspect of the present invention is a process for producing modified PAEK polymer according with the first aspect of the invention, which comprises: reacting an oxime derived from a PAEK as defined in the invention, [PAEK]=N-OH, with a compound of formula Q-(R¹)-X, wherein Q is selected from: methanesulfonyloxy, p-toluenesulfonyloxy, 2-nitrobenzenesulfonyloxy-, 4-nitrobenesulfonyloxy-, trifluoromethanesulfonyloxy, CO₂H, OH, I, Br, Cl, or a good leaving group for nucleophilic substitution reactions and R¹ and X are as defined in the first aspect of the invention.

The third aspect of the invention relates to the use of the modified PAEK as defined in the first aspect of the invention for preparing PAEK type derivatives with surfaces containing covalently bonded fluorescent molecules, polymers, peptides, proteins, or saccharides.

Fully processed PAEK elements, including medical devices can be submitted to the surface modification process of the invention and stored for long periods, before using them in further "click" conjugation reactions to incorporate biologically active components, labelling components or other compounds to the surface of the material.

Therefore a fourth aspect of the invention relates to a medical device, wherein the medical device is made of the PAEK as defined in the first aspect of the invention.

When modified PAEK polymers as defined in the first aspect of the invention are treated with osteogenic RGD peptidomimetic and/or OGP₁₀₋₁₄ peptidomimetic comprising the complementary latent "click" groups, osteointegration properties of the modified PAEK are improved.

Therefore the fifth aspect of the invention is a conjugated PAEK polymer wherein the X group of the modified PAEK polymer according the first aspect of the invention is replaced by a RGD peptidomimetic and/or OGP₁₀₋₁₄ peptidomimetic bound to a Y group wherein the Y is a group selected from: N-maleimide, N-maleimide-furan cycloadduct, thiol, thio acid, azide, sulfonylazide, ethynyl and iodoethynyl.

A sixth aspect of the invention is a process for producing a conjugated PAEK polymer according the fifth aspect of the invention which comprises reacting a PAEK polymer of the first aspect of the invention with a RGD peptidomimetic and/or OGP₁₀₋₁₄ peptidomimetic bound to a Y group wherein the Y is a group selected from: N-maleimide, N-maleimide-furan cycloadduct, thiol, thio acid, azide, sulfonylazide, ethynyl and iodoethynyl.

As previously mentioned, RGD and /or OGP₁₀₋₁₄ peptidomimetic surface treated biomaterials show enhanced osteoblast adhesion and improved osteoinduction properties. These peptidomimetics have shown to be active in promoting cell adhesion or growth.

Therefore, the seventh aspect of the invention relates to a medical device or a tissue engineering matrix or cell culture matrix comprising a PAEK polymer with a surface modified with an RGD peptidomimetic and/or OGP₁₀₋₁₄ peptidomimetic.

Fluorescence is a leading signal transduction method for the formation of chemosensory devices. Conjugated PAEK-fluorescent is a stable fluorescent conjugated polymer by the incorporation of a dansyl group to a PAEK polymer according the firs aspect of the invention.
Therefore, the eighth aspect of the invention relates to a fluorescent conjugated PAEK polymer wherein X group of the modified PAEK polymer according to the first aspect of the invention is replaced by the group with the formula:

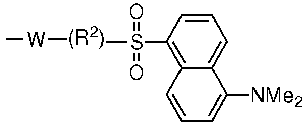

W is a biradical group selected from the groups represented by the formulae:

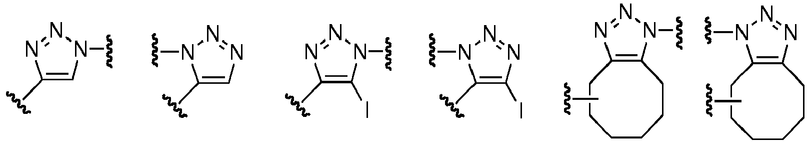

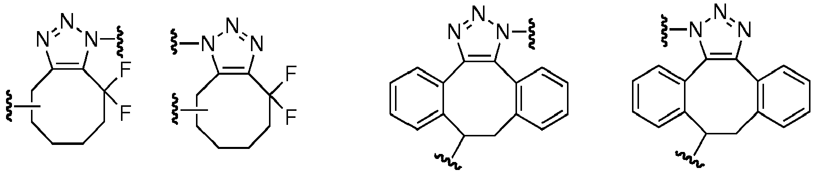

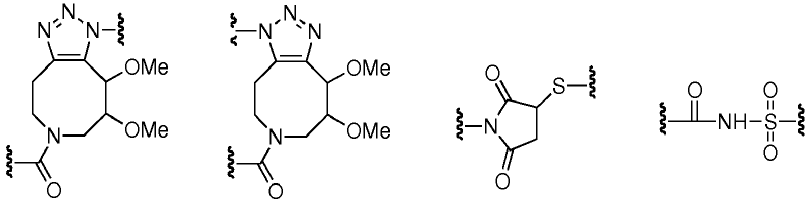

Also it is an aspect of the present invention a process for producing a conjugated PAEK polymer according the eight aspect of the invention which comprises reacting a PAEK polymer according the first aspect of the invention with a dansyl group bound to a Y group defined by the formula: 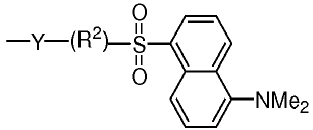 wherein Y is a group selected from: N-maleimido, N-maleimido-furan cycloadduct, thiol, thio acid, azido, sulfonylazido, ethynyl and iodoethynyl.

### BRIEF DESCRIPTION OF THE DRAWINGS

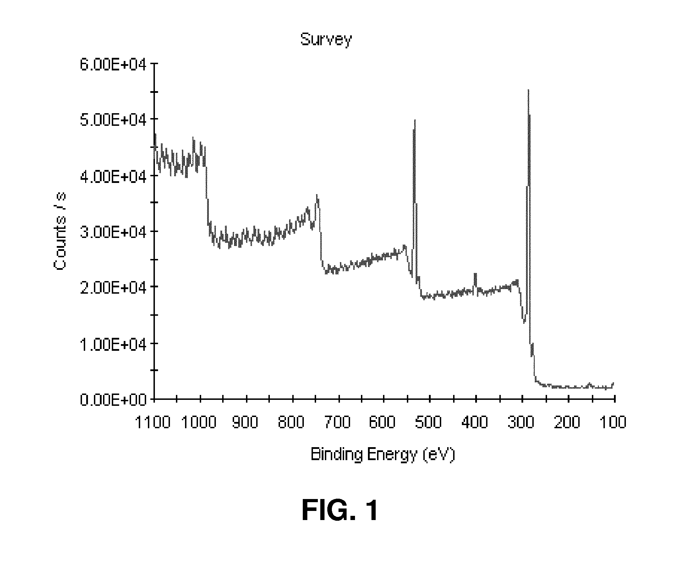

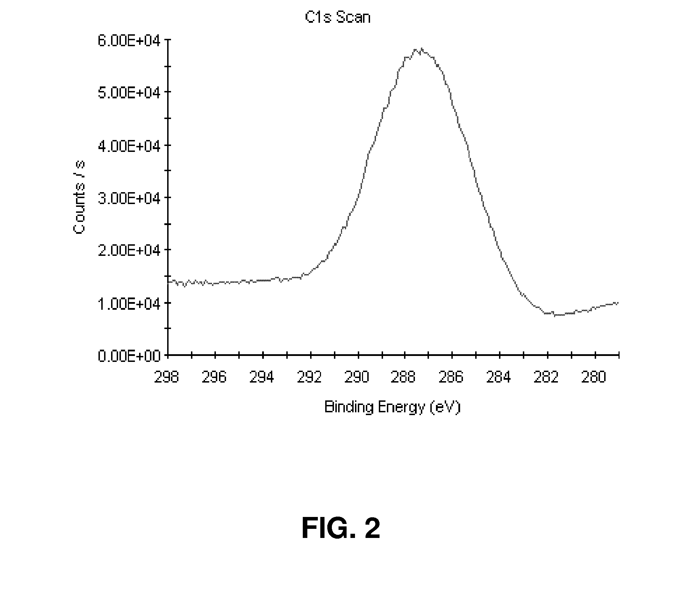

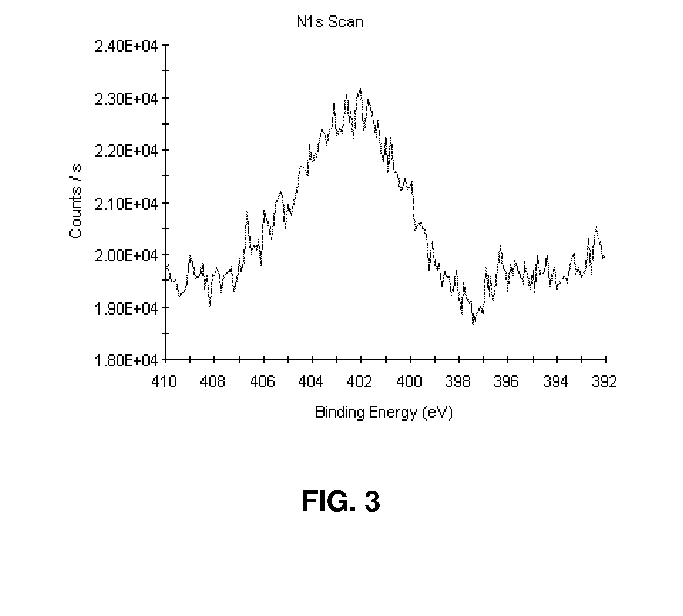

Figure 1 shows the X-ray photoelectron spectroscopy (XPS) spectrum of polished PEEK with the surface modified with a mixture of RGD and OGP₁₀₋₁₄ peptidomimetics (example 16. Figures 2, 3 and 4 show three spectra magnifications of C1s, N1 s and 01 s peaks).

### DETAILED DESCRIPTION OF THE INVENTION

The term "polyaryletherketone polymer (PAEK )" as used herein, refers to polyaryletherketone polymer (PAEK) comprising an aromatic rings with ether and ketones linkages in the backbone, wherein the aromatic ring is a substituted or unsubstituted phenylene as represented by the formula (I); 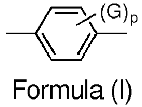 wherein G is independently in each occurrence: hydrogen, a C₁₋₄ alkyl or a halogen; more preferably G is: hydrogen, methyl, ethyl, chlorine, bromine, or fluorine; p is an integer between 0 and 4 inclusive.

As used herein, the term "alkyl" includes both saturated straight chain and branched hydrocarbon substituents. Preferably, C₁₋₂₀ alkyl groups, more preferably C₁₋₆ alkyl groups. Particularly preferred alkyl groups include, for example, methyl, ethyl, propyl, isopropyl, butyl, isobutyl, tert-butyl, pentyl and hexyl.

As used herein, the term "alkylene" includes biradical hydrocarbon saturated straight chains and branched chains attaching simultaneously two molecular fragments or functional groups. Preferably, C₁₋₂₀ alkylene groups, more preferably C₁₋₆ alkylene groups. Particularly preferred alkylene groups include, for example, methylene, ethylene, propylene and butylene.

As used herein, the term "aryl" includes substituted or unsubstituted single-ring aromatic groups in which each atom of the ring is carbon. Preferably the ring is a 5- to 7-membered ring, more preferably a 6-membered ring.

As used herein the term "medical device" means any instrument, apparatus, appliance, material or other article, whether used alone or in combination, intended to be used for the purpose of:
diagnosis, prevention, monitoring, treatment or alleviation of
disease;
diagnosis, monitoring, treatment, alleviation of or compensation for an injury or handicap;
investigation, replacement or modification of the anatomy or of a physiological process.

As used herein the term "medical device" includes stents, stent grafts, catheters, guide wires, balloons, filters (e.g., vena cava filters), vascular grafts, intraluminal paving systems, pacemakers, electrodes, leads, defibrillators, joint and bone implants, spinal implants, access ports, intra-aortic balloon pumps, heart valves, sutures, artificial hearts, neurological stimulators, cochlear implants, retinal implants, and other devices that can be used in connection with therapeutic coatings, prosthetic bone implant, endooseous implant, scaffold for bone tissue regeneration. Such medical devices are implanted or otherwise used in body structures, cavities, or lumens such as the vasculature, gastrointestinal tract, abdomen, peritoneum, airways, esophagus, trachea, colon, rectum, biliary tract, urinary tract, prostate, brain, spine, lung, liver, heart, skeletal muscle, kidney, bladder, intestines, stomach, pancreas, ovary, uterus, cartilage, eye, bone, joints, and the like. Preferably endooseous implant, scaffold for bone tissue regeneration.

As mentioned above, a first aspect of the invention refers to a modified polyaryletherketone polymer (PAEK) comprising aromatic rings with ether and ketone linkages in the backbone, wherein the aromatic ring is a substituted or unsubstituted phenylene as represented by the formula (I); 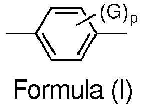 wherein G is independently in each occurrence: hydrogen, a C₁₋₄ alkyl or a halogen; more preferably G is: hydrogen, methyl, ethyl, chlorine, bromine, or fluorine; p is an integer between 0 and 4 inclusive; characterized in that in said PAEK polymer at least one ketone group is replaced by C=N-O-(R¹)-X wherein:
R¹ is a biradical consisting of C₁₋₂₀ alkylene; optionally substituted with one or more C₁₋₄ alkyl groups; optionally containing -C=C- bonds; optionally containing -C≡C-bonds; in which 0, 1, 2, 3, 4, 5 or 6 -CH₂- groups are optionally replaced by groups selected from: -O-, -S-, -N(H)-, -N(C₁₋₄ alkyl)-, -CO-, -C(O)O-, - C(O)NH-, -C(O)N(C₁₋₄ alkyl)-, -NHC(O)NH- or -NHC(O)O- and;
X is a group selected from: N-maleimide, N-maleimide-furan cycloadduct, thiol, thio acid, azide, sulfonylazide, ethynyl, iodoethynyl or an activated cyclooctynyl group represented by the formulae:

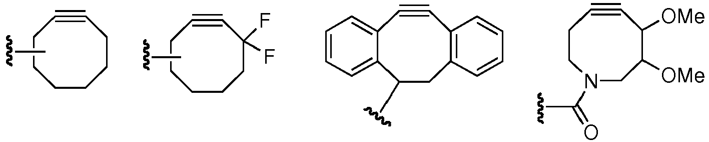

In a preferred embodiment PAEK polymer is selected from the group: poly(etherketone) (PEK), poly(etheretherketone) (PEEK), poly(etheretherketoneketone) (PEEKK), poly(etherketoneetherketoneketone) (PEKEKK), poly (oxy-p-phenylenecarbonyl-p-phenylene) and mixtures thereof. In a most preferred embodiment PAEK is PEEK. In a particularly preferred embodiment PEEK is compact PEEK or porous PEEK. In a more preferred embodiment the porous PEEK structure presents a trimodal pore distribution as follows:
A: pores of an average diameter about 50µm to 500 µm, which are interconnected throughout the whole structure,
B: voids between adjacent pores A of an average diameter about 5µm to 70 µm, and
C: pores of an average diameter about 5µm or less, which are located in the walls of the pores A and B.

In other preferred embodiment R¹ is a polyethyleneoxide group selected from -(CH₂CH₂O)ᵣ- and -(CH₂CH₂O)ᵣCH₂CH₂-; where r is an integer between 0 and 8.

As mentioned above, the second aspect of the present invention is a process for producing modified PAEK polymer according with the first aspect of the invention, which comprises: reacting an oxime derived from a PAEK as defined in the first aspect of the invention, [PAEK]=N-OH, with a compound of formula Q-(R¹)-X, wherein Q is selected from: methanesulfonyloxy, p-toluenesulfonyloxy, 2-nitrobenzenesulfonyloxy-, 4-nitrobenesulfonyloxy-, trifluoromethanesulfonyloxy, CO₂H, OH, I, Br, Cl, or a good leaving group for nucleophilic substitution reactions and R¹ and X are as defined in the first aspect of the invention.

In a preferred embodiment of the second aspect of the invention the process is carried out in the presence of a base and in the presence of a dehydrating reagent.

As mentioned above, a fifth aspect of the invention is a conjugated PAEK polymer wherein the X group of the modified PAEK polymer according the first aspect of the invention is replaced by a RGD peptidomimetic and/or a OGP₁₀₋₁₄ peptidomimetic bound to a Y group wherein the Y is a group selected from: N-maleimide, N-maleimide-furan cycloadduct, thiol, thio acid, azide, sulfonylazide, ethynyl and iodoethynyl.

The X group of the PAEK polymer take part in an addition reaction with the Y group of the peptidomimetic, to give a W group.

Therefore, in a preferred embodiment in the conjugated PAEK polymer according the fifth aspect of the invention, the X group of the modified PAEK polymer according the first aspect of the invention is replaced by a RGD peptidomimetic, wherein the RGD peptidomimetic is defined by the formula (II):

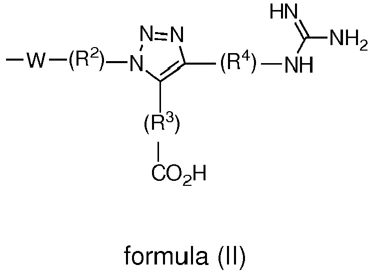

wherein:
R² is a biradical selected from C₁₋₂₀ alkylene; in which 0, 1, 2, 3, 4, 5 or 6 -CH₂- groups are optionally replaced by groups selected from: -O-, -S-, - C(O)O-, -C(O)NH-, -C(O)N(C₁₋₄alkyl)-, -NHC(O)NH-, -NHC(O)O-; and
R³ is a biradical selected from C₁₋₆ alkylene; optionally containing one or more -C=C- bonds; optionally containing -C≡C- bonds; in which 0, 1, 2 or 3 -CH₂-groups are optionally replaced by groups selected from -O- and -S-; optionally substituted with one or more groups selected from C₁₋₄ alkyl, phenyl, -F, -Cl, -OH, -O(C₁₋₄alkyl), -S(C₁₋₄alkyl), -SO₂Ph, -CN, -NO₂, -CO(C₁₋₄ alkyl), -CO₂H, -CO₂(C₁₋₄alkyl), -CONH₂, -CONH(C₁₋₄alkyl), -CON(C₁₋₄alkyl)₂; and
R⁴ is a biradical selected from C₁₋₆ alkylene; in which 0, 1, 2 or 3 -CH₂-groups are optionally replaced by groups selected from -O- and -S-; optionally substituted with one or more groups selected from C₁₋₄ alkyl, phenyl, C₆₋₁₀ aryl; and
W is a biradical group selected from the groups represented by the formulae:

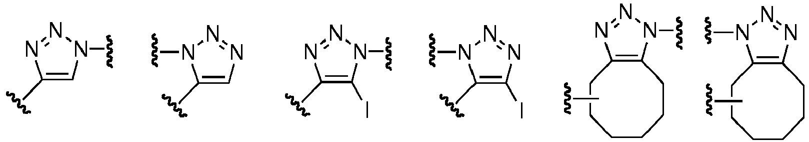

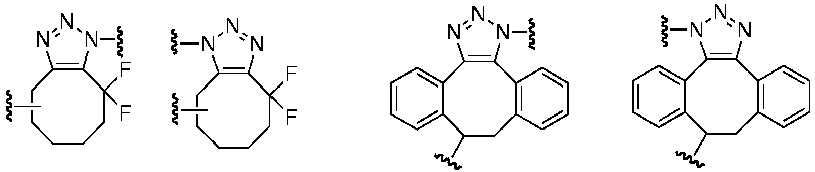

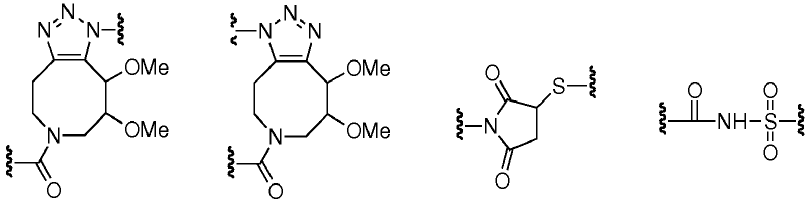

More preferably R² is -(CH₂CH₂O)ᵣCH₂CH₂-; where r is an integer between 0 and 8; R³ is CH₂CH₂- or -CH=CH-, and R⁴ is -CH₂OCH₂CH₂- or -CH₂OCH₂-.

In a preferred embodiment in the conjugated PAEK polymer according to the fifth aspect of the invention, the X group of the modified PAEK polymer according the first aspect of the invention is replaced by a OGP₁₀₋₁₄ peptidomimetic wherein the OGP₁₀₋₁₄ peptidomimetic is defined by the formula (III): 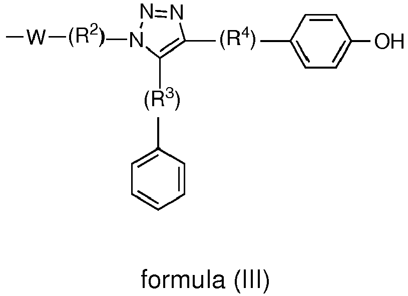 wherein:
R², R³, R⁴ and W are as defined above. More preferably R² is -(CH₂CH₂O)ᵣCH₂CH₂-; where r is an integer between 0 and 8; R³ is CH₂CH₂-or -CH=CH-, and R⁴ is -CH₂OCH₂CH₂- or -CH₂OCH₂-.

In a more preferred embodiment, in the conjugated PAEK polymer according the fifth aspect of the invention the X group of the modified PAEK polymer is replaced by the group of formula (II) and by the group of formula (III).

The process for producing a conjugated PAEK according the fifth aspect of the invention comprises reacting a PAEK polymer according the first aspect of the invention, with a compound or mixtures of compounds of the formulae: 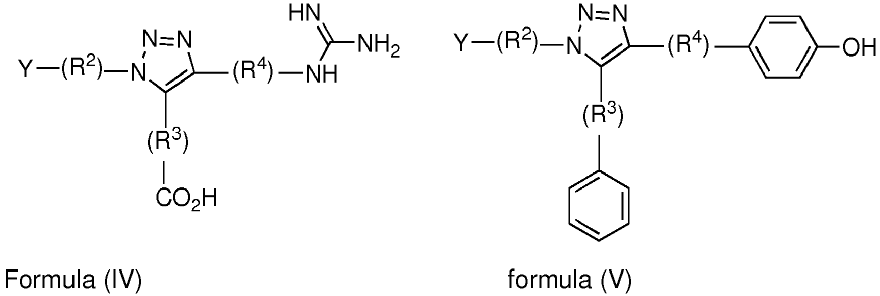 wherein:
R², R³, R⁴ and W are as above, and
Y is a group selected from N-maleimido, N-maleimido-furan cycloadduct, thiol, thio acid, azido, sulfonylazido, ethynyl and iodoethynyl.

In a preferred embodiment the process is carried out in the absence of bases and a copper salts.

### EXAMPLES

The following examples are provided for illustrative means, and are not meant to be limiting of the present invention.

Acronyms of reagents, solvents or techniques used are defined as follows:
Boc: tert-Butoxycarbonylamino group
Dansyl: 5-(Dimethylamino)naphthalene-1-sulfonyl group
DIPEA: Diisopropyletylamine
DMF: N,N-Dimethylformamide
NBS: N-Bromosuccinimide
NMR: Nuclear Magnetic Resonance
OGP: Osteogenic Growth Peptide.
PEEK: Poly-ether ether ketone
RGD: Arginine-Glycine-Aspartic acid.
THF: Tetrahydrofuran
TLC: Thin Layer Chromatography

Reactions involving PEEK compact materials were carried out in PEEK-1000 semicrystalline 20 x 20 x 5 mm size square samples from Ketron (KETRON PEEK 1000, ref. 41300000, PoliFluor S.L) and medical grade implantable PEEK CLASSIX LSG compact disk samples from Invibio (PoliFluor S.L), referred in the text as PEEK-A (raw) and PEEK-M (mechanically polished) respectively.

Reactions involving PEEK porous materials, referred in the text as PEEK-P, were carried out in disk samples (9 mm diameter/ 3 mm thin), prepared according to patent patent application ES2010/070867 (WO/2011/076971).

(Porous PEEK article as an implant) with VESTAKEEP® 2000 P powder (LATI Industria Termoplastici S. p. A.).

The contact angle values of PEEK samples, untreated or with the surface modified (I), were measured at room temperature using a computerized image analysis (DIGIDROP system). Results are collected in Table 2.

Fluorescence of PEEK samples with the surface modified with dansyl groups was measured at room temperature by using a UVIKON 922 spectrophotometer operating at an excitation wavelength of λex = 337 nm and emission wavelength of λem = 492 nm. Fluorescence was measured in relative units and results are collected in Table 3.

PEEK samples, untreated or with the surface modified were subjected to XPS analysis at room temperature under ultra-high vacuum conditions (<10⁻⁷ mbar) using a Microlab 300A instrument of Thermo Fisher equipped with magnesium Kα monochromatized radiation at 1253.6 eV anode. The measurements were done in a Constant Analyzer Energy mode (CAE) with a 200 eV pass energy for survey spectra. Results are collected in Table 4 and Figures 1- 4.

### EXAMPLE 1: Compact PEEK, porous PEEK and PEEK with the surface modified as oxime.

Raw compact PEEK-A exhibited a contact angle of 84.1º±5.0 (Table 2) and a fluorescence of 192 (Table 3). Surface XPS analysis recorded at room temperature was C 86.4%, O 13.6% for raw compact PEEK-A and C 84.3%, O 15.7% for mechanically polished compact PEEK-M (Table 4).

PEEK porous samples (PEEK-P) were prepared with a trimodal pore distribution with an average pore diameter of 120-180 µm, interconnected by voids of about 5 -70 µm between adjacent pores and a surface presenting a fine pore distribution with an average diameter of 5-10 µm or less. A contact angle value of 117.8º±1.0 (Table 2) was measured for this PEEK-P material and a fluorescence of 129.2 (Table 3). Surface XPS analysis: C 78.2%, O 21.8% (Table 4).

Compact PEEK and porous PEEK materials with the surface modified as oxime were prepared following a procedure described in Macromolecules, 1991, 24: 3045-3049.

Both porous and compact PEEK samples were cleaned by immersion in an ultrasonic bath with methanol for 30 min and subsequently dried at room temperature under reduced pressure overnight. Each set of 10 samples of porous and compact PEEK, 3.0 g of hydroxylamine hydrochloride, 10 mL of ethanol, and 2 mL water were introduced to a round-bottomed flask. Sodium hydroxide (5.5 g) was added in five portions, shaking after each addition. The balloon flask was purged with nitrogen, then heated at 40ºC for 24h, and finally refluxed for 24h. After cooling the suspension, the samples were extracted, rinsed successively with 10% aqueous HCl (5 x 30 mL) and ethanol (3 x 30 mL), and dried at room temperature.

Porous PEEK-P modified as oxime presented a contact angle value of 42.3º ± 1.0 (Table 2), and a fluorescence lower than 100 measured under identical conditions to the parent PEEK-P sample (Table 2).

PEEK-A modified as oxime presented a contact angle value of 70.7º ± 3.7 (Table 2).

### EXAMPLE 2: PEEK with the surface modified as azide. Material according to the first aspect of the invention wherein R¹=-CH₂CH₂-; X= N₃

2-(p-Toluenesulfonyloxy)ethylazide: 2-Azidoethanol (2.5 g, 28.8 mmol) was added to a solution of tosyl chloride (6.7 g, 21.6 mmol) in pyridine (15 mL) cooled to 0ºC and the mixture was stirred at such temperature for 24h. Then, CH₂Cl₂ (30 mL) was added and the solution was washed with NH₄Cl and water. The organic phase was separated, dried (MgSO₄) and evaporated to afford a colorless oil. (Yield 5.6 g, 78%). ¹H-NMR (500 MHz, CDCl₃): δ 7.80 (d, 2H, Ar), 7.40 (d, 2H, Ar), 4.25 (t, 2H, OCH₂), 3.50 (t, 2H, NCH₂), 2.50 (s, 3H, CH₃).

Compact PEEK-A oxime and porous PEEK-P oxime samples (n=10 each) prepared as described in Example 1, were introduced under nitrogen atmosphere into a flask containing a mixture of potassium carbonate (1.38 g, 10 mmol), 2-(p-toluenesulfonyloxy)ethylazide (1 g, 4 mmol) and acetone (10 mL). The suspension was stirred at 80ºC for 24 h., and the samples were washed repeatedly with water and methanol. Finally, the samples were dried at r.t. under vacuum for 5 h.

Surface XPS analysis for compact PEEK-A modified as azide: C 83.1%, O 14.3%, N 2.6% (Table 4).
Surface XPS analysis for porous PEEK-P modified as azide: C 59.6%, O 36.9%, N 3.5% (Table 4).

### EXAMPLE 3: PEEK with the surface modified as maleimide. Material according to the first aspect of the invention wherein R¹= -CH₂CH₂-; X= N-maleimide

N-(2-p-Toluenesulfonyloxyethyl)maleimide-furan cycloadduct: N-(2-Hydroxyethyl)maleimide furan cycloadduct (0.5 g, 2.4 mmol) and p-toluenesulfonyl chloride (0.72 g, 3.8 mmol) were introduced to a balloon flask under nitrogen atmosphere and were dissolved in dichloromethane (40 mL). Then pyridine (1.5 mL, 18 mmol) was added and the mixture was stirred at r.t. for 24 h. Then, the solvent was evaporated and the product was washed with a saturated solution of NaHCO₃ (20 mL x 2) and with a solution of HCl 1 M (20 mL x 2). Afterwards, the product was dissolved in dichloromethane and was evaporated. Yield: 587 mg, (67%). ¹H-NMR (500 MHz, CDCl₃): δ 7.79 (d, 2H, Ar-CH), 7.36 (d, 2H, Ar-CH), 6.54 (s, 2H, CH=CH), 5.27 (s, 2H, CH), 4.22 (t, 2H, CH₂), 3.77 (t, 2H, CH₂), 2.88 (s, 2H, CH), 2.47 (s, 3H, CH₃).

Porous PEEK oxime (PEEK-P) samples (n=2) prepared as described in Example 1, were reacted with a mixture of potassium carbonate (0.23 g), N-(2-p-toluenesulfonyloxyethyl)maleimide-furan cycloadduct (0.10 g) and acetone (2 mL). The suspension was stirred at 70ºC for 16 h., and the sample was washed repeatedly with water and methanol. Then it was sonicated in MeOH/H₂O (1:1) for 45 min. Finally, the sample was dried at 90ºC under vacuum for 72 h.

Surface characterization data for porous PEEK-P modified as maleimide. Contact angle: 140.2º ± 8.0 (Table 2). XPS analysis: C 73.9%, O 23.7%, N 2.4% (Table 4).

### EXAMPLE 4: PEEK with the surface modified as alkyne. Material according to the first aspect of the invention wherein R¹= -CH₂-; X= C≡CH

Porous PEEK oxime samples (n=2) prepared as described in Example 1, were introduced under nitrogen atmosphere into a test tube containing a mixture of potassium carbonate (0.41 g), propargyl bromide (0.16 mL) and acetone (2 mL). The suspension was stirred at 80ºC for 24 h, and the sample was washed repeatedly with water and methanol. Finally, the sample was dried at r.t. under vacuum for 5 h.

Surface XPS analysis for porous PEEK-P modified as alkyne: C 78.9%, O 19.2%, N 1.9% (Table 4).

### EXAMPLE 5: PEEK with the surface modified as cycloalkyne. Material according to the first aspect of the invention wherein: R¹= - CH₂CH₂OCH₂CH₂O-; X= cyclooctyn-3-yl

3-(6-Iodo-1,3-dioxahexyl)-cyclooctyne: To a solution of O-(cyclooctyn-3-yl)-diethylene glycol (100 mg, 0.47 mmol) in anhydrous DMF (10 mL) was added (PhO)₃PMel (0.43 g, 0.94 mmol). The resulting solution was stirred at r. t. for 30 min. Then, MeOH (1 mL) was added the mixture was evaporated at reduced pressure. The crude obtained was used without additional purification. Yield: 103.2 mg, (65%). ¹H-NMR (500 MHz, CDCl₃): δ 4.24 (t, 1H, -CH-C≡C-), 3.77-3.67 (m, 5H, O-CH₂), 3.53 (m, 1H, -CH-O), 1.91 (t, 2H, CH₂-I), 2.25-1.45 (m, 10H, -CH₂-).

Porous and compact PEEK oxime samples (n=2) prepared as described in Example 1, were introduced under nitrogen atmosphere into a test tube containing a mixture of potassium carbonate (0.41 g), 3-(6-lodo-1,3-dioxahexyl)-cyclooctyne (0.20 g) and acetone (2 mL). The suspension was stirred at 40ºC for 24 h., and the samples were washed repeatedly with water and methanol. Finally, the samples were dried at r.t. under vacuum for 5 h. Surface characterization data for porous PEEK-P modified as cycloalkyne.

Contact angle: 139.2º ±18.0 (Figure 1). XPS analysis: C 81.8%, O 17.2%, N 1.0% (Table 4).

Surface characterization data for compact PEEK-M modified as cycloalkyne. XPS analysis: C 83.3%, O 14.8%, 1.9% (Table 4).

### EXAMPLE 6: Fluorescent PEEK from azide-functionalized polymer. Material according to the eighth aspect of the invention wherein a material according the first aspect of the invention wherein R¹= -CH₂CH₂- and X=N₃ is functionalized with N-propargyldansyilamide

N-Propargyldansylamide: A solution of dansyl chloride (500 mg, 1.67 mmol), propargylamine (0.14 mL, 2.00 mmol), triethylamine (0.27 mL, 2.00 mmol) and dichloromethane (5 mL) was stirred under nitrogen atmosphere at room temperature for 15 min. Then, an aqueous phosphate buffer solution was added to adjust the pH= 7.4 and the product was extracted with dichloromethane (3 x 5mL). The organic phase was dried over MgSO₄ and evaporated to afford the pure product. Yield: 551 mg (100%). ¹H NMR (500 MHz, CDCl₃): δ 8.61 (sb, 1H, NH), 8.55 (d, 1H, Ar), 8.26 (t, 2H, Ar), 7.54 (m, 2H, Ar), 7.19 (d, 1H, Ar), 4.85 (t, 1H, NH), 3.77 (dd, 2H, N-CH₂), 2.90 (s, 6H, CH₃), 1.92 (t, 1H, CCH).

Porous PEEK-azide (n=1) and compact PEEK-azide (n=1) samples prepared as described in Example 2 ( R¹= -CH₂CH₂-; X= N₃), were suspended in a test tube containing 1 mL of a solution of CuSO₄ in THF/ H₂O 1:1 (2 mg/mL). Then, N-propargyldansylamide (20 mg, 0,063 mmol), 1 mL of a solution of sodium ascorbate in THF/ H₂O 1:1 (8 mg/ mL) and an additional 8 mL of THF/H₂O 1:1 (purged with N₂) were added successively to the solution and the mixture was stirred for 24 hours at room temperature. Then, the samples were repeatedly washed with water and THF until no residual fluorescence could be detected in the washing liquid and the samples were finally dried under vacuum at room temperature for 5 h.

Surface characterization data for fluorescent compact PEEK-A from azide-functionalized polymer. Fluorescence: 188.6 (Table 3). XPS analysis: C 94.0%, O 4.2%, N 1.6%, S 0.2% (Table 4).

Surface characterization data for fluorescent porous PEEK-P from azide-functionalized polymer. Fluorescence: 959.2 (Table 3). XPS analysis: C 70.8%, O 24.1%, N 3.7%, S 1.4% (Table 4).

### EXAMPLE 7: Fluorescent PEEK from maleimide-functionalized polymer. Material according to the eighth aspect of the invention wherein a material according the first aspect of the invention wherein R¹= -CH₂CH₂- and X= N-maleimido is functionalized with N,N'-Bisdansylcistine diethyl ester

N,N'-Bisdansylcistine diethyl ester: Dansyl chloride (0.5 g, 1.85 mmol) was added under nitrogen atmosphere to a solution of cystin diethyl ester (0.34 g, 1.35 mmol) and triethylamine (0.77 mL, 5.55 mmol) in dry dichloromethane (20 mL) and the resulting mixture was stirred at room temperature for 24h. Then, EtOAc (150 mL) was added and the organic phase was washed successively with aqueous saturated solutions of NH₄Cl (2 x 25 mL) and NaCl (1 x 30 mL). Drying (Na₂SO₄) and evaporation of the solvents in vacuum afforded the crude product, which was purified by column chromatography (silica gel; EtOAc/ hexanes 1:1). Yield: 464 mg (33%). ¹H NMR (500 MHz, CDCl₃): δ 8.61-8.25 (3d, 6H, Ar-Dansyl), 7.65 (2t, 2H, Ar-Dansyl), 7.25 (d, 2H, Ar-Dansyl), 5.75 (d, 2H, NH), 4.25 (m, 2H, NCHCO), 3.75 (m, 4H, OCH₂), 3.25-3.00 (dd, 4H, -SCH₂), 2.95 (s, 12H, (CH₃)₂), 1.00 (t, 6H, CH₃).

N-Dansyl cysteine ethyl ester: N,N'-bisdansylcistine diethyl ester (90 mg, 0.12 mmol) was dissolved in a mixture of dry THF (5 mL) and MeOH (0.5 mL) under nitrogen atmosphere. The solution was cooled at 0ºC, NaBH₄ (45 mg, 0.63 mmol) was added and the resulting mixture was stirred for 1h at room temperature. Upon completion (TLC, EtOAc/hexanes 1:1) the solvent was removed under reduced pressure and EtOAc (50 mL) was added to the residue. The organic layer was washed with aqueous saturated NH₄Cl (3 x 15 mL), dried (Na₂SO₄) and the solvent was evaporated to afford the pure product. Yield: 65 mg (100%). ¹H NMR (500 MHz, CDCl₃): δ 8.61 (d, 1H, Ar-Dansyl), 8.40 (m, 2H, Ar-Dansyl), 7.65 (m, 2H, Ar-Dansyl), 7.25 (d, 1H, Ar-Dansyl), 5.75 (d, 1H, NH), 4.95 (as, 1H, SH), 4.25 (m, 1H, NCHCO), 4.00 (m, 2H, OCH₂), 3.60 (dd, 1H, -SCH), 2.95 (s, 6H, (CH₃)₂), 2.50 (dd, 1H, -SCH), 1.05 (t, 3H, CH₃).

A porous PEEK-maleimide sample (n=1) prepared as described in Example 3, ( R¹= -CH₂CH₂-; X= N-maleimido) was suspended in a test tube containing dry THF (1.5 mL), Et₃N (10 µL) and N-dansyl cysteine ethyl ester (14 mg). The resulting mixture was stirred at room temperature under nitrogen atmosphere for 16h. Then, the PEEK sample was repeatedly washed with THF and MeOH until no residual fluorescence could be detected in the washing liquid and the sample was finally dried under vacuum at room temperature for 3 h.

Surface characterization data for fluorescent porous PEEK-P from maleimide-functionalized polymer. Fluorescence: 765.4 (Table 3). XPS analysis: C 73.4%, O 23.6%, N 2.5%, S 0.5% (Table 4).

### EXAMPLE 8: Fluorescent PEEK from alkyne-functionalized polymer. Material according to the eighth aspect of the invention wherein a material according the first aspect of the invention wherein R¹= -CH₂- and X= C≡CH is functionalized with 2-Azidoethyl dansylate

2-Azidoethyl dansylate: A solution of dansyl chloride (500 mg, 1.67 mmol), 2-azidoethanol (278 mg, 4 mmol), Et₃N (0.54 mL, 4 mmol) and dichloromethane (5 mL) was stirred under nitrogen atmosphere at room temperature overnight. The reaction mixture was washed with aqueous phosphate buffer solution (pH= 7.4) (2 x 3 mL) and the product was extracted with dichloromethane (3 x 5mL). The combined organic phases were dried over MgSO₄ and evaporated to afford pure 2-azidoethyl dansylate. Yield: 0.55 g (99%). ¹H NMR (500 MHz, CDCl₃): δ 8.66 (d, 1H, Ar-Dansyl), 8.32 (d, 2H, Ar-Dansyl), 7.60 (m, 2H, Ar-Dansyl), 7.26 (d, 1H, Ar-Dansyl), 4.14 (t, 2H, -CH₂), 3.47 (t, 2H, -CH₂), 2.92 (s, 6H, (CH₃)₂).

A porous PEEK-alkyne sample (n=1) prepared as described in Example 4 (R¹= -CH₂-; X= C≡CH), was suspended in a test tube containing 1 mL of a solution of CuSO₄ in THF/ H₂O 1:1 (2 mg/mL). Then, 2-azidoethyl dansylate (20 mg, 0.05 mmol), 1 mL of a solution of sodium ascorbate in THF/ H₂O 1:1 (8 mg/ mL) and an additional 8 mL of THF/H₂O 1:1 (purged with N₂) were added successively to the solution and the mixture was stirred for one day at room temperature. Then, the sample was repeatedly washed with water and THF until no residual fluorescence could be detected in the washing liquid and the sample was finally dried under vacuum at room temperature for 3 h.

Surface characterization data for fluorescent porous PEEK-P from alkyne-functionalized polymer. Fluorescence: 1000 (Table 3). XPS analysis: C 68.5%, O 25.6%, N 4.5%, S1.4% (Table 4).

### EXAMPLE 9: Fluorescent PEEK from cycloalkyne-functionalized polymer. Material according to the eighth aspect of the invention wherein a material according the first aspect of the invention wherein R¹= -CH₂CH₂OCH₂CH₂O-and X= cyclooctyn-3-yl is functionalized with 2-Azidoethyl dansylate

Porous PEEK oxime samples (n=2) functionalized as cycloalkyne ( R¹= - CH₂CH₂OCH₂CH₂O-; X= cyclooctyn-3-yl) prepared as described in Example 5, were introduced under nitrogen atmosphere into a test tube containing 2-azidoethyl dansylate (50 mg, 0.13 mmol), in 2 mL of a THF/H₂O 1:1 mixture and the suspension was stirred at 40ºC for 24 h. The sample was repeatedly washed with water and THF until no residual fluorescence could be detected in the washing liquid and the sample was finally dried under vacuum at room temperature for 5 h.

Surface characterization data for fluorescent porous PEEK from cycloalkyne-functionalized polymer. Fluorescence: > 1000 (Table 3).

### EXAMPLE 10: Porous PEEK-P with the surface modified as RGD mimetic. Material according the fifth aspect of the invention wherein in the formula (II) R¹= -CH₂CH₂OCH₂CH₂O- ,R²=R³= -CH₂CH₂-; R⁴= -CH₂OCH₂CH₂-; W= 4,5-bicyclooctene-1,2,3-triazole

4-(4-tert-Butoxycarbonylamino-2-oxa-butyl)-1-(2-hydroxyethyl)-5-iodo-1,2,3-triazole: To a stirred solution of Cul (218 mg, 1.15 mmol) in dried CH₃CN, NBS (223 mg, 1.25 mmol), 2-(tert-butoxycarbonylamino)ethyl propargyl ether (208 mg, 1.04 mmol), 2-azidoethanol (100 mg, 1.15 mmol) and DIPEA (200 µl, 1.15 mmol) were added. The mixture was stirred at room temperature for two hours. The solvent was evaporated, the residue was dissolved in CH₂Cl₂, washed with 10% aqueous Na₂S₂O₃ and the organic phase was dried (MgSO₄) and evaporated. The product was purified by column chromatography (silica gel; EtOAc/hexanes 1:1). Yield: 300 mg (70%). ¹H NMR (500 MHz, CDCl₃) δ 4.63 (s, 2H), 4.54 - 4.48 (t, J = 4.9 2H), 4.18 (t, *J* = 5.0, 2H), 3.62 (t, J= 5.1, 2H), 3.34 (t, J = 5.0, 2H), 1.46 (s, 9H).

4-(4-tert-Butoxycarbonylamino-2-oxa-butyl)-1-(2-hydroxyethyl)-5-(2-methoxycarbonyl-ethyl)-1,2,3-triazole: A suspension of 4-(4-tert-butoxycarbonylamino-2-oxa-butyl)-1-(2-hydroxyethyl)-5-iodo-1,2,3-triazole (500 mg, 1.21 mmol), Pd(OAc)₂ (27,2 mg, 0.121 mmol) and NaHCO₃ (254,7 mg, 3,03 mmol) in anhydrous DMF (5 mL) was prepared in a flame-dried flask under nitrogen atmosphere. Methyl acrylate (273.1 µL, 3,03 mmol) was added and the mixture was stirred at 85ºC overnight. The solvent was evaporated and the product was purified by column chromatography (silica gel, EtOAc/hexanes 1:1). This intermediate product (355 mg, 0.96 mmol) was dissolved in dry MeOH and ammonium formate (302.2 mg, 4.8 mmol) and 10%-Pd-C (107.8 mg, 0.096 mmol) were added. The mixture was refluxed overnight. The product was purified by filtration over celite. Yield: 330 mg (74%). ¹H NMR (500 MHz, CDCl₃): δ 4.55 (s, 2H), 4.40 (t, *J=* 4.7, 2H), 4.06 (t, *J* = 4.7, 2H), 3.64 (s, 3H), 3.54 (t, *J* = 5.1, 2H), 3.26 (s, 2H), 3.05 (t, *J* = 7.3, 2H), 2.68 (t, *J* = 7.4, 2H), 1.41 (s, 9H).

1-(2-Azidoethyl)-4-(4-tert-butoxycarbonylamino-2-oxa-butyl)-5-(2-carboxyethyl)-1,2,3-triazole: To a stirred solution of 4-(4-tert-butoxycarbonylamino-2-oxa-butyl)-1-(2-hydroxyethyl)-5-(2-methoxycarbonylethyl)-1,2,3-triazole (135 mg, 0.36 mmol), cooled to 0ºC in dried CH₂Cl₂ was added triphenyl phosphine (190.2 mg, 0.73 mmol) and NBS (129.0 mg, 0.73 mmol). The mixture was stirred over one hour. The solvent was evaporated and the crude product was dissolved in dried DMF. Then, NaN₃ (94.3 mg, 1.45 mmol) and Nal (54.3 mg, 0.36 mmol) were added and the mixture was stirred at room temperature over 48 hours. After evaporation of the solvent, the crude product was dissolved in THF/H₂O (1:1) and LiOH•H₂O (151.9mg, 3.62 mmol) was added and the mixture was stirred for 8 hours. Then, the solvent was evaporated and the product was purified by acid and basic extraction with CH₂Cl₂. Yield: 143.9 mg (70%). ¹H NMR (500 MHz, CD₃OD) δ 4.64 (s, 2H), 4.57 (t, *J =* 5.5, 2H), 3.88 (t, *J =* 5.5, 2H), 3.54 (t, *J =* 5.5, 2H), 3.25 (t, *J =* 5.3, 2H), 3.12 (t, *J =* 7.3, 2H), 2.73 (t, *J =* 7.2, 2H), 1.45 (s, 9H).

1-(2-Azidoethyl)-5-(2-carboxyethyl)-4-(4-N-guanidyl-2-oxa-butyl)-1,2,3-triazole (compound of formula (IV); R²= R³= -CH₂CH₂-; R⁴= -CH₂OCH₂CH₂-; Y= N₃): A 6M HCl solution in dioxane (2 mL, 12 mmol) was added to 1-(2-azidoethyl)-4-(4-tert-butoxycarbonylamino-2-oxa-butyl)-5-(2-carboxyethyl)-1,2,3-triazole (80 mg, 0.21 mmol) and the mixture was stirred at room temperature over 1 h. Then, the solvent was evaporated, the crude amine hydrochloride was dissolved in methanol (5 mL) and sodium bicarbonate was added until pH=7. Amino(imino)methanesulfonic acid (31.0 mg, 0.25 mmol) was added, the mixture was stirred at room temperature over 1 h. and it was evaporated to dryness. The residue was extracted with MeOH (3 x 5 mL) and the solution was evaporated at reduced pressure. Yield (90 %). ¹H NMR (500 MHz, D₂O): δ 4.62 (s, 1H), 4.46 (t, *J* = 5.0, 1H), 3.97 (t, *J* = 5.0, 1H), 3.68 (t, *J* = 4.8, 1H), 3.38 - 3.33 (m, 1H), 3.00 (t, *J* = 7.6, 1H), 2.43 (t, *J =* 7.6, 1H).

Porous PEEK oxime samples (n=2) functionalized as cycloalkyne prepared as described in Example 5 (R¹= -CH₂CH₂OCH₂CH₂O-; X= cyclooctyn-3-yl), were introduced under nitrogen atmosphere into a test tube containing a 2 mL of a THF/H₂O 1:1 mixture. Then, 0.2 mg of compound 1-(2-Azidoethyl)-5-(2-carboxyethyl)-4-(4-N-guanidyl-2-oxa-butyl)-1,2,3-triazoleof formula (IV): (R²= R³= -CH₂CH₂-; R⁴= -CH₂OCH₂CH₂-; Y= N₃) were added and the suspension was stirred at 40ºC for 24 h. The sample was washed repeatedly with a solution of HCl 0.1M, an aqueous solution of NH₃ (pH=11), water and methanol and dried at room temperature under vacuum for 5 h.
Surface XPS analysis for porous PEEK-P modified with RGD mimetic: C 79.4%, O 18.7% N 1.9% (Table 4).

### EXAMPLE 11: Compact PEEK-M with the surface modified as RGD mimetic. Material according the fifth aspect of the invention wherein in the formula (II) R¹= -CH₂CH₂OCH₂CH₂O-; R²=R³= -CH₂CH₂-; R⁴= -CH₂OCH₂CH₂-; W= 4,5-bicyclooctene-1,2,3-triazole

Compact PEEK-M oxime samples (n=2) functionalized as cycloalkyne prepared as described in Example 5 (R¹= -CH₂CH₂OCH₂CH₂O-; X= cyclooctyn-3-yl), was reacted with 0.2 mg of RGD mimetic of formula (IV): (R²=R³= -CH₂CH₂-; R⁴= -CH₂OCH₂CH₂-; Y= N₃ prepared as described in Example 10). Reaction conditions and purification were identical to the Example 10.

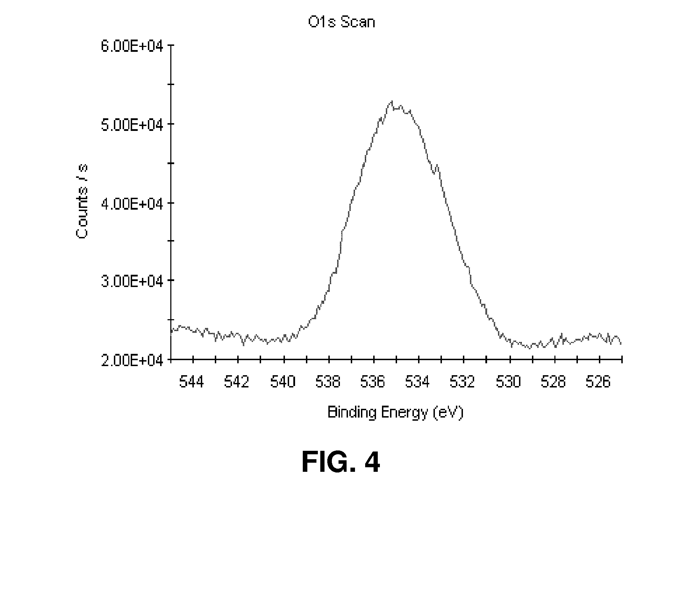

Surface characterization data for polished compact PEEK-M modified with RGD mimetic. XPS analysis: C 84.4%, O 13.3%, N 2.3% (Table 3 and Figure 4).

### EXAMPLE 12: Compact PEEK-M with the surface modified as RGD mimetic. Material according the fifth aspect of the invention wherein in the formula (II) wherein R¹= -CH₂CH₂OCH₂CH₂O-; R²= -[CH₂CH₂O]₃CH₂CH₂HN(CO)OCH₂CH₂-; R³= -CH₂CH₂-; R⁴= -CH₂OCH₂CH₂-; W= 4,5-bicyclooctene-1,2,3-triazole

1-(16-Azido-5-aza-3,8,11,14-tetraoxa-4-oxohexadecyl)-4-(4-tert-butoxycarbonylamino-2-oxa-butyl)-5-(2-methoxycarbonylethyl)-1,2,3-triazole: In a flame-dried flask, 4-(4-tert-butoxycarbonylamino-2-oxa-butyl)-1-(2-hydroxyethyl)-5-(2-methoxycarbonyl-ethyl)-1 ,2,3-triazole (0.27 mmol, 100 mg), prepared as described in Example 10, was dissolved in dry THF (2 mL) under nitrogen atmosphere and after addition of DIPEA (0.54 mmol, 96 µL), the mixture was cooled to 0ºC. Subsequently, a solution of triphosgene (0.17 mmol, 49 mg) was added dropwise and then the mixture was allowed to reach the room temperature during 30 min. The suspension was filtered through a celite pad and the solvent was evaporated under pressure to obtain the intermediate chloroformate, which was immediately dissolved in dry CH₂Cl₂ (2 mL). DIPEA (0.54 mmol, 93 µL) and 8-azido-3,6-dioxaoctylamine (0.27 mmol, 60 mg) were added and the mixture was kept stirring overnight. The product was purified by column chromatography using CH₂Cl₂/MeOH 90/10 as eluent. Yield: 85 mg (51 %). IR (cm⁻¹): 3339, 2869, 2102 (N₃), 1704 (C=O), 1522 (tri). ¹H NMR (500 MHz, CDCl₃) δ 4.61 (s, 2H, OCH₂C=Ctri), 4.56 (t, *J =* 5.1, 2H, N1triCH₂CH₂), 4.49 (t, *J =* 5.0, 2H, N1triCH₂CH₂), 3.67 (s, 3H, COOCH₃), 3.67-3.51 (m, 14H, OCH₂CH₂NHBoc and OCH₂CH₂O), 3.40 - 3.28 (m, 6H, OCH₂CH₂NHBoc, OCH₂CH₂NHCOO and OCH₂CH₂N₃), 3.04 (t, J *=* 7.6, 2H, CH₂CH₂CO₂CH₃), 2.70 (t, *J =* 7.2, 2H, CH₂CH₂CO₂CH₃), 1.43 (s, 9H, tBu).

1-(16-Azido-5-aza-3,8,11,14-tetraoxa-4-oxohexadecyl)-5-(2-carboxyethyl)-4-(4-N-guanidyl-2-oxa-butyl)-1,2,3-triazole (compound of formula (IV); R²= - [CH₂CH₂O]₃CH₂CH₂HN(CO)OCH₂CH₂-; R³= -CH₂CH₂-; R⁴= -CH₂OCH₂CH₂-; Y= N₃): A 4M HCl solution in dioxane (2 mL, 8 mmol) was added to 1-(16-azido-5-aza-3,8,11,14-tetraoxa-4-oxohexadecyl)-4-(4-tert-butoxycarbonylamino-2-oxa-butyl)-5-(2-methoxycarbonylethyl)-1,2,3-triazole (75 mg, 0.14 mmol) and the mixture was stirred at room temperature over 2 h. Then, the solvent was evaporated, the crude amine hydrochloride (45 mg, 0.08 mmol) was dissolved in methanol (5 mL) and potassium carbonate was added until slightly basic pH. Amino(imino)methanesulfonic acid (11 mg, 0.09 mmol) was added, the mixture was stirred at room temperature over 1 h. Lithium hydroxide (4.0 mg, 0.09 mmol) was added and the solution was stirred for 4h. Upon completion, the solids were filtered off with MeOH (3 x 5 mL), the solution was evaporated at reduced pressure and the resulting crude product was purified by preparative reverse phase HPLC (C18 column, MeCN:H₂O 80:20). Yield (75 %). ¹H NMR (500 MHz, D₂O) δ 4.63 (m, 4H, OCH₂C=Ctri and N1triCH₂CH₂), 4.46 (m, 2H, N1triCH₂CH₂), 3.70-3.60 (m, 12H), 3.50 (m, 2H, OCH₂CH₂NHCOO), 3.44 (t, *J* = 4.2, 2H, OCH₂CH₂N₃), 3.36 (t, *J* = 4.7, 2H, OCH₂CH₂NHCOO), 3.18 (m, 2H, CH₂CH₂guanidine), 3.02 (t, *J* = 7.4, 2H, CH₂CH₂CO₂H), 2.45 (t, *J=* 7.5, 2H, CH₂CH₂CO₂H).

Compact PEEK-M oxime samples (n=2) functionalized as cycloalkyne prepared as described in Example 5( R¹= -CH₂CH₂OCH₂CH₂O-; X= cyclooctyn-3-yl), was reacted with 0.2 mg of RGD mimetic of formula (IV): (R²= -[CH₂CH₂O]₃CH₂CH₂HN(CO)OCH₂CH₂-; R³= -CH₂CH₂-; R⁴= - CH₂OCH₂CH₂-; Y= N₃ prepared as described above). Reaction conditions and purification were identical to the Example 10.

Surface characterization data for polished compact PEEK-M modified with RGD mimetic. XPS analysis: C 82.2%, O 14.6%, N 3.2% (Table 4).

### EXAMPLE 13: Estimation of RGD surface concentration in a compact PEEK-M modified with RGD mimetic. Material according the fifth aspect of the invention wherein in the formula (II) wherein R¹= -CH₂CH₂OCH₂CH₂O-; R²=R³= -CH₂CH₂-; R⁴= -CH₂OCH₂CH₂-; W= 4,5-bicyclooctene-1,2,3-triazole

The RGD-modified PEEK-M sample was prepared as described in Example 11.

Enzyme-linked immunosorbent assay (ELISA) test (n=3) afforded a concentration of 0.169 ± 0.043 pmol x cm-2 for a RGD mimetic concentration during the functionalization of 0.1 mg x mL-1 vs. 0.082 ± 0.031 pmol x cm-2 in the PEEK surfaces modified as oxime samples prepared as described in Example 1, and 0.00 ± 0.00 pmol x cm-2 in the untreated control PEEK samples.

### EXAMPLE 14: Compact PEEK-M with the surface modified as OGP mimetic. Material according the fifth aspect of the invention wherein in the formula (III) wherein R¹= -CH₂CH₂OCH₂CH₂O-; R²= -CH₂CH₂-; R³= -CH=CH-; R⁴= - CH₂OCH₂-; W= 4,5-bicyclooctene-1,2,3-triazole

1-(2-Hydroxyethyl)-5-iodo-4-[2-oxa-3-(4-triisopropylsilyloxyphenyl)-proyl]-1,2,3-triazole: To a stirred solution of Cul (401 mg, 2.11 mmol) in dried CH₃CN, NBS (409 mg, 2.30 mmol), 4-triisopropylsilyloxy-benzyl propargyl ether (610 mg, 1.92 mmol), 2-azidoethanol (100 mg, 1.15 mmol) and DIPEA (367 µl, 2.11 mmol) were added. The mixture was stirred at room temperature for two hours. The solvent was evaporated, the residue was dissolved in CH₂Cl₂, washed with 10% aqueous Na₂S₂O₃ and the organic phase was dried (MgSO₄) and evaporated. The product was purified by column chromatography (silica gel; EtOAc/hexanes 1:1). Yield: 315 mg (31%). ¹H NMR (500 MHz, CDCl₃) δ 7.26 (d, *J =* 8.0, 2H), 6.88 (d, *J =* 8.1, 2H), 4.61 (s, 2H), 4.54 (s, 2H), 4.49 (t, *J* = 4.2, 2H), 4.17 (d, *J* = 3.3, 2H), 1.33 - 1.23 (m, 3H), 1.12 (ds, 18H).

1-(2-Hydroxyethyl)-5-[(E)-(2-phenyl)vinyl]-4-[2-oxa-3-(4-triisopropylsilyloxyphenyl)-proyl]-1,2,3-triazole: A suspension of 1-(2-hydroxyethyl)-5-iodo-4-[2-oxa-3-(4-triisopropylsilyloxyphenyl)-proyl]-1,2,3-triazole (270 mg, 0.52 mmol), E-2-phenylvinylboronic acid (119.8 mg, 0.79 mmol), bis(triphenylphosphine) palladium (II) dichloride (14.7 mg, 0.02 mmol) and potassium hydroxide (58.8 mg, 1.05 mmol) were dissolved in anhydrous THF (4 mL) and kept at 75ºC during 2 h. The product was purified by column chromatography (silica gel, EtOAc/hexanes 1:1). Yield: 330 mg (92%). ¹H NMR (500 MHz, CDCl₃) δ 7.49 (d, *J =* 7.2, 2H), 7.41 (t, *J =* 7.3, 2H), 7.39 - 7.34 (m, 1H), 7.31 (d, *J =* 13.2, 1H), 7.26 (d, *J =* 8.4, 2H), 6.98 (d, *J =* 16.3, 1H), 6.86 (d, *J =* 8.4, 2H), 4.72 (s, 2H), 4.58 (s, 2H), 4.51 - 4.46 (m, 2H), 4.18 (dd, *J =* 10.2, 5.4, 2H), 1.32 - 1.22 (m, 3H), 1.11 (d, *J =* 7.4, 18H).

1-(2-Azidoethyl)-5-[(E)-(2-phenyl)vinyl]-4-[2-oxa-3-(4-triisopropylsilyloxyphenyl)-proyl]-1,2,3-triazole: In a dried flask cyanuric chloride (55.93 mg, 0.30 mmol) and DMF (61.06 µL) were warmed at 25 °C for 10 min. After the formation of a white solid, CH₂Cl₂ (0.5 mL) was added, followed by 1-(2-hydroxyethyl)-5-[(E)-(2-phenyl)vinyl]-4-[2-oxa-3-(4-triisopropylsilyloxyphenyl)-proyl]-1,2,3-triazole (70 mg, 0.14 mmol). The mixture was kept at room temperature during one hour and the solvent was evaporated. The resulting crude product was dissolved again in acetone, sodium azide (89.71 mg, 1.38 mmol) was added and the mixture was stirred for 24 hours. Then, cesium fluoride (300 mg, 2.0 mmol) was added, and the resulting crude was evaporated and purified by column chromatography (silica gel, EtOAc/hexanes 1:1). ¹H NMR (500 MHz, CDCl₃) δ 7.50 (d, *J =* 7.2, 2H), 7.47 - 7.33 (m, 4H), 7.33 - 7.25 (m, 3H), 6.95 (d, *J =* 16.3, 1H), 6.87 (d, *J* = 8.2, 2H), 4.75 (s, 2H), 4.71 (t, *J* = 6.4, 2H), 4.60 (s, 2H), 4.01 (t, *J* = 6.4, 2H).

Compact PEEK oxime samples (n=2) functionalized as cycloalkyne prepared as described in Example 5 (R¹= -CH₂CH₂OCH₂CH₂O-; X= cyclooctyn-3-yl), was reacted with 0.2 mg of OGP mimetic of formula (V): (R²= -CH₂CH₂-; R³= -CH=CH-; R⁴= -CH₂OCH₂-; Y= N₃, prepared as described above). Reaction conditions and purification was identical to the Example 10.
Surface characterization data for polished compact PEEK-M modified with OGP mimetic. XPS analysis: C 75.6%, O 21.2%, N 3.2% (Table 4).

### EXAMPLE 15: Compact PEEK-M with the surface modified with OGP peptidomimetics. Material according the fifth aspect of the invention wherein in the formula (III), R¹= -CH₂CH₂OCH₂CH₂O-; R²= -[CH₂CH₂O]₃CH₂CH₂HN(CO)OCH₂-; R³= -CH=CH-; R⁴= -CH₂OCH₂-; W= 4,5-bicyclooctene-1,2,3-triazole

1-(16-Azido-5-aza-3,8,11,14-tetraoxa-4-oxohexadecyl)-5-[(E)-(2-phenyl)vinyl]-4-[2-oxa-3-(4-triisopropylsilyloxyphenyl)-proyl]-1,2,3-triazole: In a flame-dried flask, 1-(2-hydroxyethyl)-5-[(E)-(2-phenyl)vinyl]-4-[2-oxa-3-(4-triisopropylsilyloxyphenyl)-proyl]-1,2,3-triazole (0.20 mmol, 100 mg) prepared as shown in Example 14, was dissolved in dry THF (1.5 mL) under nitrogen atmosphere and after addition of DIPEA (0.39 mmol, 69 µL), the mixture was cooled to 0ºC. Subsequently, a solution of triphosgene (0.12 mmol, 36 mg) was added dropwise and then the mixture was allowed to reach the room temperature during 30 minutes. The suspension was filtered through a celite pad and the solvent was evaporated under pressure to obtain the intermediate chloroformate [¹H NMR (500 MHz, CDCl₃) δ 7.50 (d, *J =* 7.3, 2H), 7.45 - 7.35 (m, 4H), 7.26 (d, *J =* 8.0, 2H), 6.92 (d, *J =* 16.4, 1H), 6.86 (d, *J =* 7.9, 2H), 4.80-4.70 (m, 6H), 4.59 (s, 2H), 1.31 - 1.21 (m, 3H), 1.11 (d, *J =* 7.3, 18H)].

The product was dissolved in dry CH₂Cl₂ (1.5 mL) and sequentially DIPEA (0.39 mmol, 69 µL) and tetraethylene glycol (0.20 mmol, 43 mg) were added and the mixture was kept stirring overnight. The product was purified by column chromatography using CH₂Cl₂/MeOH 90/10 as eluent. Yield: 116 mg (78%). IR (cm⁻¹): 2944, 2866, 2102 (N₃), 1720 (C=O), 1509. ¹H NMR (500 MHz, CDCl₃) δ 7.47 (d, *J =* 7.7, 2H), 7.42 - 7.21 (m, 6H), 6.92 (d, *J =* 16.3, 1H), 6.85 (d, *J* = 8.0, 2H), 4.72 (s, 2H), 4.64 (t, *J* = 4.8, 2H), 4.57 (s, 2H), 4.48 (s, 2H), 3.69 - 3.49 (m, 11 H), 3.45 - 3.41 (m, 2H), 3.39 - 3.32 (m, 2H), 3.28 (t, *J =* 10.9, 2H), 1.30 - 1.19 (m, 3H), 1.09 (d, *J =* 7.4, 18H).
1-(16-Azido-5-aza-3,8,11,14-tetraoxa-4-oxohexadecyl)-5-[(E)-(2-phenyl)vinyl]-4-[2-oxa-3-(4-hydroxyphenyl)-proyl]-1,2,3-triazole: A suspension of 1-(16-azido-5-aza-3,8,11,14-tetraoxa-4-oxohexadecyl)-5-[(E)-(2-phenyl)vinyl]-4-[2-oxa-3-(4-triisopropylsilyloxyphenyl)-proyl]-1,2,3-triazole (0.14 mmol, 105 mg) and cesium fluoride (0.70 mmol, 106 mg) in methanol (1.5 mL) was stirred at

room temperature for one hour. Then the solvent was evaporated and the product was purified by column chromatography using CH₂Cl₂/MeOH 95:5 as eluent. Yield: 50 mg (60%). IR(cm⁻¹): 3328, 2867, 2101 (N₃), 1705 (C=O), 1517. ¹H NMR (500 MHz, CDCl₃) δ 7.47 (d, *J=* 7.4, 2H), 7.44 - 7.25 (m, 5H), 7.20 (dd, *J =* 17.2, 8.0, 2H), 6.86 (d, *J =* 16.6, 1H), 6.80 (d, *J =* 8.1, 2H), 4.75 (s, 2H), 4.64 (d, *J* = 4.8, 2H), 4.57 (s, 2H), 4.47 (s, 2H), 3.58 (ddd, *J =* 35.4, 14.6, 6.2, 10H), 3.39 - 3.35 (m, 2H), 3.35 - 3.27 (m, 2H), 3.21 (d, *J* = 4.8, 2H). PEEK-M oxime discs (n=1) functionalized as cycloalkyne prepared as described in Example 5 (R¹= -CH₂CH₂OCH₂CH₂O-; X= cyclooctyn-3-yl), were introduced under nitrogen atmosphere into a test tube containing 1 mL of THF/H₂O 1:1 mixture, 0.1 mg of OGP mimetic (V) (R²= - [CH₂CH₂O]₃CH₂CH₂HN(CO)OCH₂CH₂-; R³= -CH=CH-; R⁴= -CH₂OCH₂-; Y= N₃) prepared as above. The suspension was stirred at 40ºC for 24 h. The discs were washed repeatedly with THF, water and methanol and dried at room temperature under vacuum for 5 h.
Surface XPS analysis for the polished PEEK-M modified with OGP mimetic: C 79.8%, O 17.7% N 2.5% (Table 4).

### EXAMPLE 16: Compact PEEK-M with the surface modified with a combination of RGD and OGP peptidomimetics. Material according the fifth aspect of the invention wherein in the formula (II) : R¹= -CH₂CH₂OCH₂CH₂O-; R²=R³= - CH₂CH₂-; R⁴= -CH₂OCH₂CH₂-; W= 4,5-bicyclooctene-1,2,3-triazole and in the formula (III) R¹= -CH₂CH₂OCH₂CH₂O-; R²= -[CH₂CH₂O]₃CH₂CH₂HN(CO)OCH₂CH₂-; R³= -CH=CH-; R⁴= -CH₂OCH₂-; W= 4,5-bicyclooctene-1,2,3-triazole

PEEK-M oxime discs (n=2) functionalized as cycloalkyne prepared as described in Example 5 (R¹= -CH₂CH₂OCH₂CHO₂-; X= cyclooctyn-3-yl), were introduced under nitrogen atmosphere into a test tube containing 2 mL of THF/H₂O 1:1 mixture, 0.1 mg of RGD mimetic of formula (IV) (R²= R³= - CH₂CH₂-; R⁴= -CH₂OCH₂CH₂; Y= N₃) prepared as described in Example 10, and 0.1 mg of OGP mimetic of formula (V) (R²= -CH₂CH₂-; R³= -CH=CH-; R⁴= -CH₂OCH₂-; Y= N₃) prepared as described in Example 10. The suspension was stirred at 40ºC for 24 h. The discs were washed repeatedly with a solution of HCl 0.1M, an aqueous solution of NH₃ (pH= 11), water and methanol and dried at room temperature under vacuum for 5 h.

Surface XPS analysis for PEEK-M: C 84.3%, O 15.7% N 0%; for PEEK-M (I) functionalized as cycloalkyne: C 83.3%, O 14.8% N 1.9%; for PEEK-M (I) modified with RGD and OGP: C 74.9%, O 21.6% N 3.59% (Figures 1-4).

### EXAMPLE 17: Compact PEEK-M with the surface modified with a combination of RGD and OGP peptidomimetics. Material according the fifth aspect of the invention wherein in the formula (II): R¹= -CH₂CH₂OCH₂CH₂O-; R²=R³= - CH₂CH₂-; R⁴= -CH₂OCH₂CH₂-; W= 4,5-bicyclooctene-1,2,3-triazole and in the formula (III) R¹= -CH₂CH₂OCH₂CH₂O-; R²= -[CH₂CH₂O]₃CH₂CH₂HN(CO)OCH₂CH₂-; R³= -CH=CH-; R⁴= -CH₂OCH₂-; W= 4,5-bicyclooctene-1,2,3-triazole.

PEEK-M oxime discs (n=2) functionalized as cycloalkyne prepared as described in Example 5(R¹= -CH₂CH₂OCH₂CH₂O-; X= cyclooctyn-3-yl), were introduced under nitrogen atmosphere into a test tube containing 2 mL of THF/H2O 1:1 mixture, 0.1 mg of RGD mimetic of formula (IV) (R²= - [CH₂CH₂O]₃CH₂CH₂HN(CO)OCH₂CH₂-; R³= -CH₂CH₂-; R⁴= -CH₂OCH₂CH₂-; Y= N₃) prepared as described in Example 11, and 0.1 mg of OGP mimetic (V) (R²= -[CH₂CH₂O]₃CH₂CH₂HN(CO)OCH₂CH₂-; R³= -CH=CH-; R⁴= - CH₂OCH₂-; Y= N₃) prepared following the method described in Example 10. The suspension was stirred at 40ºC for 24 h. The discs were washed repeatedly with a solution of HCl 0.1 M, an aqueous solution of NH₃ (pH=11), water and methanol and dried at room temperature under vacuum for 5 h.

### EXAMPLE 18: Compact PEEK-M with the surface modified with a combination of Cl and Br-containing groups.

13-Azido-1-(4-chlorophenyl)-2,5,8,11-tetraoxatridecane: Potassium hydroxide (26 mg, 0.46 mmol) was flame-dried in a round-bottomed flask under nitrogen atmosphere. Then a solution of 1-azido-11-hydroxy-3,6,9-trioxaundecanol (50 mg, 0.23 mmol) in dry THF (1.20 mL) and sodium iodide (catalytic) were added. The suspension was sonicated during 5 minutes and finally, 4-chlorobenzyl chloride (73 mg, 0.46 mmol) was added. The mixture was stirred overnight at 40ºC. After evaporation of the solvent, the product was purified by column chromatography (silica gel, EtOAc/hexanes 1:1). Yield: 67 mg (85%). IR (cm⁻¹): 2864, 2097 (N₃), 1087. ¹H NMR (500 MHz, CDCl₃) δ 7.35 - 7.25 (m, 4H), 4.54 (s, 2H), 3.70 - 3.66 (m, 14H), 3.64 (td, *J* = 4.1, 1.0, 3H), 3.38 (t, *J* = 5.1, 2H).

13-Azido-1-(4-bromophenyl)-2,5,8,11-tetraoxatridecane: Potassium hydroxide (26 mg, 0.46 mmol) was flame-dried in a round-bottomed flask under nitrogen atmosphere. Then a solution of 1-azido-11-hydroxy-3,6,9-trioxaundecanol (50 mg, 0.23 mmol) in dry THF (1.20 mL) and sodium iodide (catalytic) were added. The suspension was sonicated during 5 minutes and finally, 4-bromobenzyl chloride (94 mg, 0.46 mmol) was added. The mixture was stirred overnight at 40ºC. After evaporation of the solvent, the product was purified by column chromatography (silica gel, EtOAc/hexanes 1:4). Yield: 88 mg (90%). IR (cm⁻¹): 2864, 2096 (N₃), 1094. ¹H NMR (500 MHz, CDCl₃) δ 7.47 (d, *J =* 8.2, 1H), 7.23 (d, *J* = 8.3, 1H), 4.52 (s, 2H), 3.73 - 3.61 (m, 14H), 3.38 (t, *J =* 4.8, 2H).

PEEK-M oxime discs (n=2) functionalized as cycloalkyne of formula (I) (R¹= - CH₂CH₂OCH₂CH₂O-; X= cyclooctyn-3-yl) prepared as described in Example 5, were introduced under nitrogen atmosphere into a test tube containing 2 mL of THF/H₂O 1:1 mixture, 0.3 mg of 13-azido-1-(4-chlorophenyl)-2,5,8,11-tetraoxatridecane, and 0.3 mg of 13-azido-1-(4-bromophenyl)-2,5,8,11-tetraoxatridecane prepared as described above. The suspension was stirred at 40ºC for 24 h. The discs were washed repeatedly with THF, water and methanol and dried at room temperature under vacuum for 5 h.

Surface XPS analysis for PEEK-M functionalized with an 1:1 combination of Cl and Br containing azides: C 82.7%, Cl 0.2%, Br 0.3%, O 14.4%, N 2.4% (Table 4).

**Table 1. Table 1 shows the PEEK modifications in the examples**

| **Example nº (PEEK type)** | **PEEK (Modification)** |
|---|---|
| (PEEK-P) | 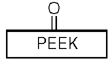 |
| (PEEK-A) | |
| (PEEK-M) | |
| 2 (PEEK-P) | 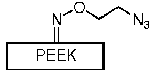 |
| 2 (PEEK-A) | |
| 3 (PEEK-P) | 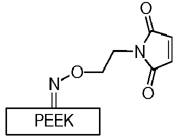 |
| 4 (PEEK-P) | 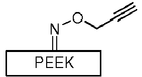 |
| 5 (PEEK-P) | 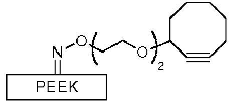 |
| 5/12 (PEEK-M) | |
| 6 (PEEK-P) | 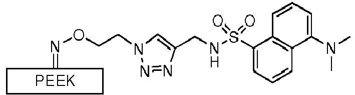 |
| 6 (PEEK-A) | |
| 7 (PEEK-P) | 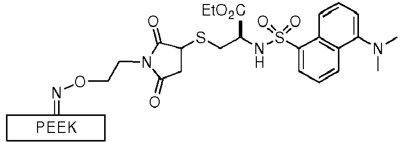 |
| 8 (PEEK-P) | 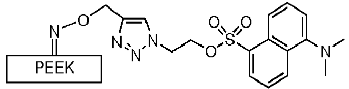 |
| 9 (PEEK-P) | 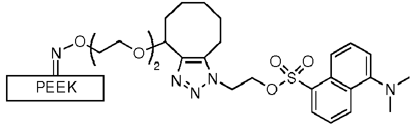 |
| 10 (PEEK-P) | 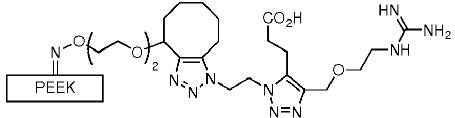 |
| 11 (PEEK-M) | 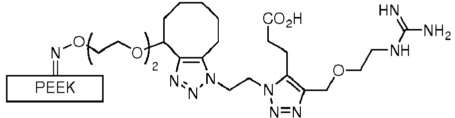 |
| 12 (PEEK-M) | 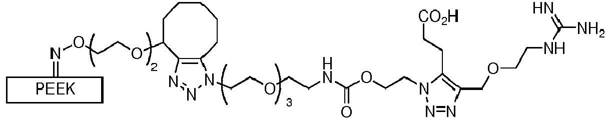 |
| 14 (PEEK-M) | 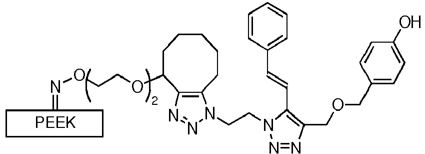 |
| 15 (PEEK-M) | 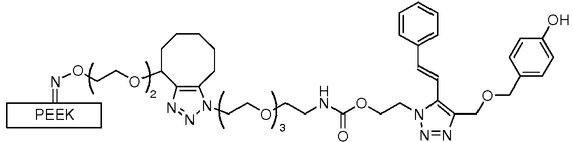 |
| 16 (PEEK-M) | 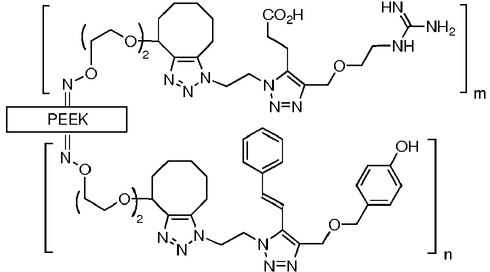 |
| 17 (PEEK-M) | 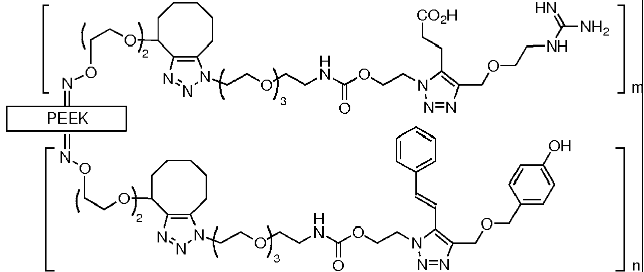 |
| 18 (PEEK-M) | 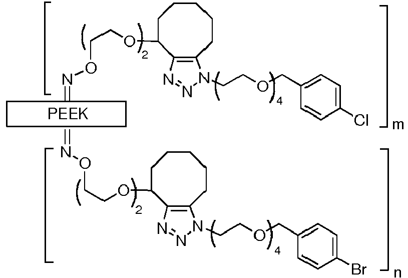 |

**Table 2. Table 2 shows contact angle values for selected PEEK samples modified at the surface. Measurements were performed by computerized image analysis (DIGIDROP system) in a range from 2 to 160º with an error of ± 0.5º.**

| **Example nº (PEEK type)** | **PEEK (Modification)** | **Contact angle** |
|---|---|---|
| PEEK-P (porous) | 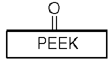 | 117.8 ± 1.0 |
| PEEK-A (compact raw) | | 84.1 ± 5.0 |
| 1 (PEEK-P) | 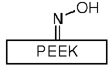 | 42.3 ± 1.0 |
| (PEEK-A) | | 70.7 ± 3.7 |
| 3 (PEEK-P) | 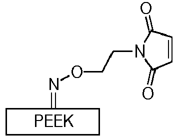 | 140.2 ± 8.0 |
| 5 (PEEK-P) | 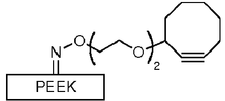 | 139.2 ± 1.0 |

**Table 3. Table 3 shows relative fluorescence values for selected PEEK samples modified at the surface with dansyl groups. Measurements were conducted using a UVIKON 922 spectrophotometer operating at an excitation wavelength of λex = 337 nm and emission wavelength of λem = 492 nm.**

| **Example nº (PEEK type)** | **PEEK (Modification)** | **Fluorescence** |
|---|---|---|
| PEEK-P (porous) | 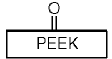 | 129.2 |
| PEEK-A (compact raw) | | 192 |
| 1 (PEEK-P) | 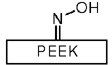 | <100 |
| 6 (PEEK-P) | 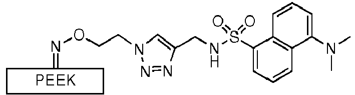 | 959.2 |
| 6 (PEEK-A) | | 188.6 |
| 7 | 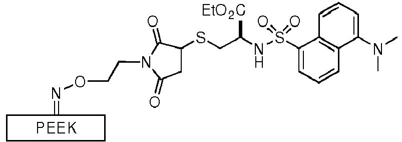 | 765.4 |
| 8 | 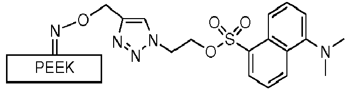 | 1000 |
| 9 | 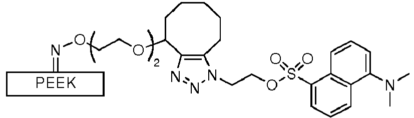 | >1000 |

**Table 4. Table 4 shows surface atom percentage values measured by X-ray photoelectron spectroscopy (XPS) for selected PEEK samples modified at the surface. Measurements were conducted under ultra-high vacuum conditions (<10-7 mbar) using a Microlab 300A of Thermo Fisher instrument equipped with magnesium Kα monochromatized radiation at 1253.6 eV anode. The measurements were conducted in a Constant Analyser Energy mode (CAE) with 200 eV pass energy for survey spectra.**

| **Example nº (PEEK type)** | **XPS (%Atom)** | | | |
|---|---|---|---|---|
| | **C** | **O** | **N** | **S** |
| (PEEK-P) | 78.2 | 21.8 | --- | --- |
| (PEEK-A) | 86.4 | 13.6 | --- | --- |
| (PEEK-M) | 84.3 | 15.7 | --- | --- |
| 2 (PEEK-P) | 59.6 | 36.9 | 3.5 | --- |
| 2 (PEEK-A) | 83.1 | 14.3 | 2.6 | --- |
| 3 (PEEK-P) | 73.9 | 23.7 | 2.4 | --- |
| 4 (PEEK-P) | 78.9 | 19.2 | 1.9 | --- |
| 5 (PEEK-P) | 81.8 | 17.2 | 1.0 | --- |
| 5/12 (PEEK-M) | 83.3 | 14.8 | 1.9 | --- |
| 6 (PEEK-P) | 70.8 | 24.1 | 3.7 | 1.4 |
| 6 (PEEK-A) | 94.0 | 4.2 | 1.6 | 0.2 |
| 7 (PEEK-P) | 73.4 | 23.6 | 2.5 | 0.5 |
| 8 (PEEK-P) | 68.5 | 25.6 | 4.5 | 1.4 |
| 10 (PEEK-P) | 79.4 | 18.7 | 1.9 | --- |
| 11 (PEEK-M) | 84.4 | 13.3 | 2.3 | --- |
| 12 (PEEK-M) | 82.2 | 14.6 | 3.2 | --- |
| 14 (PEEK-M) | 75.6 | 21.2 | 3.2 | --- |
| 15 (PEEK-M) | 79.8 | 17.7 | 2.5 | --- |
| 16 (PEEK-M) | 74.9 | 21.6 | 3.5 | --- |
| 17 (PEEK-M) | --- | --- | --- | --- |
| 18 (PEEK-M) | 82.7 | 14.4 | 2.4 | Cl 0.2 |
| | | | | Br 0.3 |

## Claims

1. A modified polyaryletherketone polymer (PAEK) comprising aromatic rings with ether and ketone linkages in the backbone, wherein the aromatic ring is a substituted or unsubstituted phenylene as represented by the formula (I); 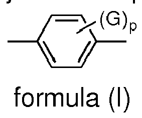 wherein G is independently in each occurrence: hydrogen, a C₁₋₄ alkyl, or a halogen; more preferably G is: hydrogen, methyl, ethyl, chlorine, bromine, or fluorine; p is an integer between 0 and 4 inclusive;
**characterized in that** in said PAEK polymer at least one ketone group is replaced by C=N-O-(R₁)-X wherein:
R¹ is a biradical consisting of C₁₋₂₀ alkylene; optionally substituted with one or more C₁₋₄ alkyl groups; optionally containing -C=C- bonds; optionally containing -C≡C- bonds; in which 0, 1, 2, 3, 4, 5 or 6 -CH₂- groups are optionally replaced by groups selected from :-O-, -S-, -N(H)-, -N(C₁₋₄ alkyl)-, -CO-, -C(O)O-, -C(O)NH-, -C(O)N(C₁₋₄ alkyl)-, -NHC(O)NH- or -NHC(O)O- and;
X is a group selected from: N-maleimide, N-maleimide-furan cycloadduct, thiol, thio acid, azide, sulfonylazide, ethynyl, iodoethynyl or an activated cyclooctynyl group represented by the formulae:

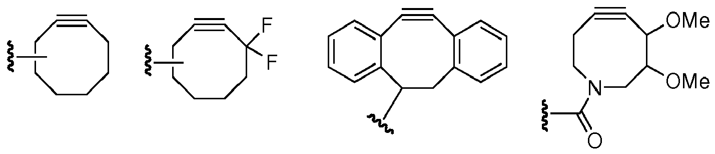

2. The modified PAEK according to claim 1 wherein the PAEK polymer is selected from the group: poly(etherketone) (PEK), poly(etheretherketone) (PEEK), poly(etheretherketoneketone) (PEEKK), poly(etherketoneetherketoneketone) (PEKEKK), and mixtures thereof.

3. The modified PAEK according to claims 1-2 wherein R¹ is a polyethyleneoxide groups selected from -(CH₂CH₂O)ᵣ- and - (CH₂CH₂O)ᵣCH₂CH₂-; where r is an integer between 0 and 8.

4. The modified PAEK according to claims 1-3 wherein the PAEK presents a porous structure.

5. The modified PAEK according to claim 4 wherein the PAEK presents a porous structure with trimodal pore distribution as follows:
A: pores of an average diameter about 50µm to 500 µm, which are interconnected throughout the whole structure,
B: voids between adjacent pores A of an average diameter about 5µm to 70 µm, and
C: pores of an average diameter about 5µm or less, which are located in the walls of the pores A and B.

6. A process for producing modified PAEK polymer according to any of the claims 1-5, which comprises:
reacting an oxime derived from a PAEK as defined according to claim 1, [PAEK]=N-OH, with a compound of formula Q-(R¹)-X, wherein Q is selected from: methanesulfonyloxy, p-toluenesulfonyloxy, 2-nitrobenzenesulfonyloxy-, 4-nitrobenesulfonyloxy-, trifluoromethanesulfonyloxy, CO₂H, OH, I, Br, Cl, or a good leaving group for nucleophilic substitution reactions and R¹ and X are as defined according to claim 1.

7. The process according to claim 6 , wherein the process is carried out in the presence of a base; and in the presence of a dehydrating reagent.

8. A conjugated PAEK polymer wherein the X group of the modified PAEK polymer according to any of the claims 1-5 is replaced by a RGD peptidomimetic and/or OGP₁₀₋₁₄ peptidomimetic bound to a Y group wherein the Y is a group selected from: N-maleimide, N-maleimide-furan cycloadduct, thiol, thio acid, azide, sulfonylazide, ethynyl and iodoethynyl.

9. The conjugated PAEK polymer according to claim 8 wherein the X group of the modified PAEK polymer according to any of the claims 1-5 is replaced by RGD peptidomimetic and the RGD peptidomimetic is defined by the formula (II): 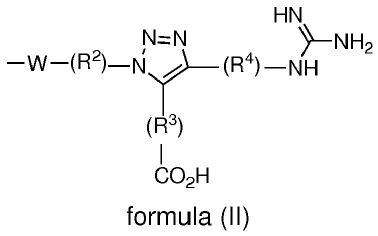 wherein:
R² is a biradical selected from C₁₋₂₀ alkylene; in which 0, 1, 2, 3, 4, 5 or 6 -CH₂- groups are optionally replaced by groups selected from: -O-, -S-, - C(O)O-, -C(O)NH-, -C(O)N(C₁₋₄alkyl)-, -NHC(O)NH-, -NHC(O)O-; and
R³ is a biradical selected from C₁₋₆ alkylene; optionally containing one or more -C=C- bonds; optionally containing -C≡C- bonds; in which 0, 1, 2 or 3 -CH₂-groups are optionally replaced by groups selected from -O- and -S-;
optionally substituted with one or more groups selected from C₁₋₄ alkyl, phenyl, -F, -Cl, -OH, -O(C₁₋₄alkyl), -S(C₁₋₄alkyl), -SO₂Ph, -CN, -NO₂, -CO(C₁₋₄ alkyl), -CO₂H, -CO₂(C₁₋₄alkyl), -CONH₂, -CONH(C₁₋₄alkyl), -CON(C₁₋₄alkyl)₂; and
R⁴ is a biradical selected from C₁₋₆ alkylene; in which 0, 1, 2 or 3 -CH₂-groups are optionally replaced by groups selected from -O- and -S-;
optionally substituted with one or more groups selected from C₁₋₄ alkyl, phenyl, C₆₋₁₀ aryl; and
W is a biradical group selected from the groups represented by the formulae:

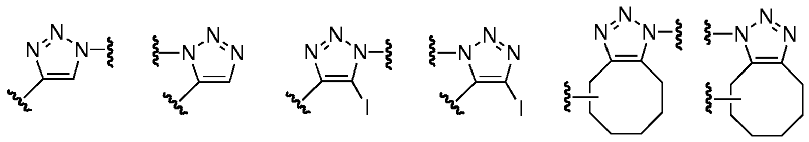

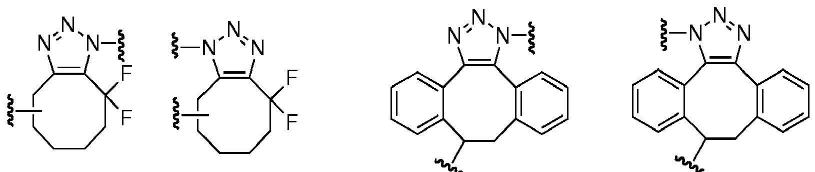

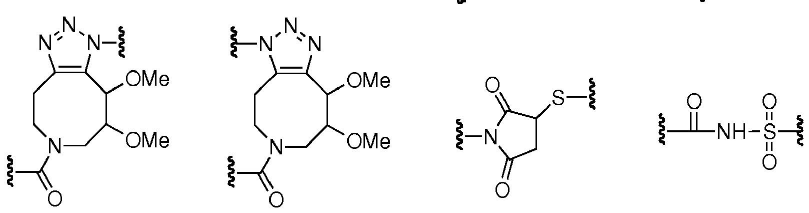

10. The conjugated PAEK of claim 9 wherein R² is -(CH₂CH₂O)ᵣCH₂CH₂-; where r is an integer between 0 and 8; R³ is CH₂CH₂- or -CH=CH-, and R⁴ is -CH₂OCH₂CH₂- or -CH₂OCH₂-

11. The conjugated PAEK polymer of claim 8 wherein X group of the modified PAEK polymer according to any of the claims 1-5 is replaced by OGP₁₀₋₁₄ peptidomimetic and the OGP₁₀₋₁₄ peptidomimetic is defined by the formula (III): 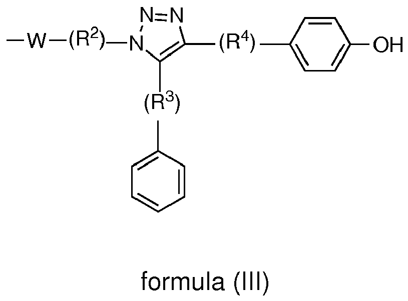 wherein:
R², R³, R⁴ and W are as defined in claim 9.

12. The conjugated PAEK of claim 11, wherein R², R³, R⁴ and W are as defined in claim 9.

13. The conjugated PAEK polymer of claim 9 wherein the X group of the modified PAEK polymer according to any of the claims 1-5 is replaced by the group of formula (III) as defined in claim 11.

14. A process for producing a conjugated PAEK polymer according to claim 8 which comprises reacting a PAEK polymer according to any of the claims 1-5 with a RGD peptidomimetic and/or OGP₁₀₋₁₄ peptidomimetic bound to a Y group wherein Y is a group selected from: N-maleimido, N-maleimido-furan cycloadduct, thiol, thio acid, azido, sulfonylazido, ethynyl and iodoethynyl .

15. A process for producing a conjugated PAEK polymer according to any of the claims 9-13 which comprises reacting a PAEK polymer according to claims 1-5, with a compound or mixtures of compounds of the formulae: 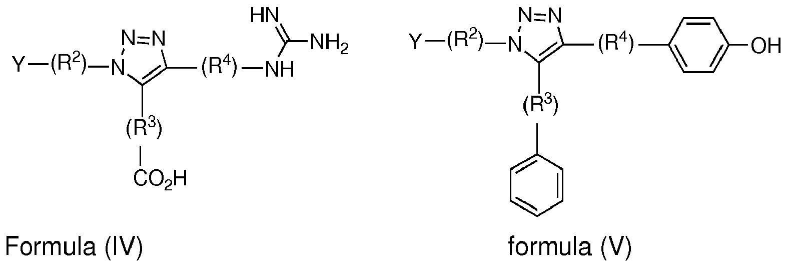 wherein:
R², R³, R⁴ and W are as defined in claim 9, and
Y is a group selected from N-maleimido, N-maleimido-furan cycloadduct, thiol, thio acid, azido, sulfonylazido, ethynyl and iodoethynyl.

16. The process of claim 15 wherein the process is carried out in the absence of a base and a copper salt.

17. A medical device made of the modified PAEK according claims 1-5.

18. A fluorescent conjugated PAEK polymer wherein the X group of the modified PAEK polymer according to any of the claims 1-5 is replaced by the group with the formula 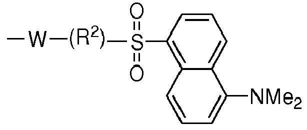 wherein W is as defined is claim 9.

19. A process for producing a conjugated PAEK polymer according to claim 18 which comprises reacting a PAEK polymer according to any of the claims 1-5 with a dansyl group bound to a Y group defined by the formula: 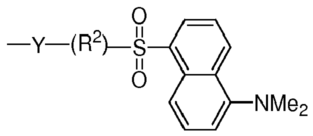 wherein Y is a group selected from: N-maleimido, N-maleimido-furan cycloadduct, thiol, thio acid, azido, sulfonylazido, ethynyl and iodoethynyl.

20. A medical device or a tissue engineering matrix or cell culture matrix comprising the polymer defined as defined in any of the claims 8-13 or 18.
